# EUROPEAN PATENT APPLICATION

(11) **EP 4 249 918 A1**
(43) Date of publication of application: **27.09.2023**
(21) Application number: 23163596.2
(22) Date of filing: 22.03.2023
(51) Int. Cl.: G01N 33/72

(54) **SAMPLE ANALYZER, SAMPLE TESTING METHOD, AND GLYCATED HEMOGLOBIN TESTING METHOD**

(30) Priority: 22.03.2022 CN 202210283533; 22.03.2022 CN 202210283613
(71) Applicant: Shenzhen Mindray Bio-Medical Electronics Co., Ltd., Shenzhen, Guangdong 518057 (CN)
(72) Inventor: SUN, Ziling, Shenzhen, 518057 (CN); YU, Qi, Shenzhen, 518057 (CN); DU, Shaoqing, Shenzhen, 518057 (CN)
(74) Representative: KIPA AB

(57) **Abstract**

The disclosure relates to a sample analyzer, a sample testing method, and a glycated hemoglobin testing method. The sample analyzer includes: a sample preparation apparatus configured to aspirate a blood sample from a container containing a test blood sample of a subject, and prepare a first pretreatment test solution by using the aspirated blood sample and a pretreatment reagent; a first test apparatus configured to test the first pretreatment test solution to obtain intermediate measurement information of the first pretreatment test solution; a controller configured to select a test mode to be performed from the plurality of different test modes based on the intermediate measurement information, and control the sample preparation apparatus to prepare a test sample solution by using a second pretreatment test solution and a test reagent according to the selected test mode to be performed; and a second test apparatus configured to test the test sample solution to obtain a test result for the test blood sample for a target test item. Sample retesting is thus avoided as much as possible.

## Description

### TECHNICAL FIELD

The disclosure relates to the field of in vitro diagnostics, and in particular to, a sample analyzer, a sample testing method, and a glycated hemoglobin testing method.

### BACKGROUND

In a sample test, such as a glycated hemoglobin test, a sample measurement result may exceed an effective measurement linear range, which will lead to an abnormal sample measurement result.

In an existing solution, usually, after a measurement result of a sample is obtained, it is determined whether the measurement result of the sample exceeds an effective measurement range. If the measurement result of the sample exceeds the effective measurement range, the sample is retested in another test mode. However, frequent retesting results in reduced testing efficiency and increased cost.

### SUMMARY

In order to solve the above technical problems, an object of the disclosure is to provide a sample analyzer, a sample testing method, and a glycated hemoglobin testing method, which can expand the linear range of detection of glycated hemoglobin, that is, the effective measurement range, thereby avoiding sample retesting as much as possible.

In order to achieve the object, a first aspect of the disclosure provides a sample analyzer, including:
a sample preparation apparatus configured to aspirate a blood sample from a container containing a test blood sample of a subject, and prepare a first pretreatment test solution by using the aspirated blood sample and a pretreatment reagent;
a first test apparatus configured to test the first pretreatment test solution, so as to obtain intermediate measurement information of the first pretreatment test solution;
a controller configured to select a test mode to be performed from a plurality of different test modes based on the intermediate measurement information, and control the sample preparation apparatus to prepare a test sample solution by using a second pretreatment test solution and a test reagent according to the selected test mode to be performed, wherein the second pretreatment test solution is prepared by the sample preparation apparatus using the test blood sample of the subject and a/the pretreatment reagent; and
a second test apparatus configured to test the test sample solution, so as to obtain a test result of the test blood sample for a target test item.

In this way, before preparing the test sample solution to be finally tested by the second test apparatus, an intermediate test solution produced in the preparation process of the test sample solution, particularly, a pretreatment test solution obtained after pretreatment of the test blood sample, is tested to obtain intermediate measurement information, and a test mode for preparing the test sample solution is selected based on the intermediate measurement information, so that the test sample solution is prepared according to the selected test mode suitable for the current test blood sample. Thus, an accurate test result can be obtained through one test, an effective test range of one test can be improved, and retesting of the test blood sample can be reduced or even avoided, thereby improving the overall testing efficiency of the sample analyzer and reducing the overall testing cost.

A second aspect of the disclosure provides a sample analyzer, including:
a sample preparation apparatus configured to prepare test sample solutions respectively corresponding to at least two different test modes based on a test blood sample, wherein each test sample solution is prepared from a pretreatment test solution and a test reagent, and the pretreatment test solution is prepared from the test blood sample and a pretreatment reagent;
a first test apparatus configured to test at least one pretreatment test solution, so as to obtain intermediate measurement information of the at least one pretreatment test solution;
a second test apparatus configured to test the test sample solutions respectively corresponding to the test modes, so as to obtain a plurality of test results for a same target test item; and
a controller configured to: based on the intermediate measurement information of the at least one pretreatment test solution, select at least one test result from the plurality of test results, and output the at least one test result.

In this way, the plurality of test results for the same detection item are obtained, and a test result to be output is selected based on the intermediate measurement information, which can reduce or even avoid retesting of the test blood sample.

A third aspect of the disclosure provides a sample analyzer, including:
a sample preparation apparatus configured to prepare, from a test blood sample of a subject, a test sample solution for testing glycated hemoglobin;
an information obtaining apparatus configured to obtain erythrocyte-related information of the subject;
a second test apparatus configured to test the test sample solution, so as to obtain a glycated hemoglobin test result; and
a controller configured to:
   when an initial test of a glycated hemoglobin item is performed on the test blood sample of the subject, select a test mode to be performed from a plurality of different glycated hemoglobin test modes based on the erythrocyte-related information of the subj ect,
   control the sample preparation apparatus to prepare the test sample solution according to the selected test mode to be performed, wherein the test sample solution is prepared based on the test blood sample of the subject, a pretreatment reagent, and a test reagent for testing glycated hemoglobin, and
   control the second test apparatus to test the test sample solution.

In this way, when the initial test of the glycated hemoglobin item is performed on the test blood sample of the subject, the glycated hemoglobin test mode for preparing the test sample solution is selected based on the erythrocyte-related information of the subject. Thus, an accurate glycated hemoglobin test result can be obtained through one test, an effective test range of one test can be improved, and retesting of the glycated hemoglobin item can be reduced or even avoided, thereby improving the testing efficiency of the glycated hemoglobin item and reducing the overall testing cost.

A fourth aspect of the disclosure provides a sample analyzer, including:
a sample preparation apparatus configured to, under at least two different glycated hemoglobin test modes, prepare test sample solutions corresponding to the respective glycated hemoglobin test modes based on a test blood sample of a subject;
an information obtaining apparatus configured to obtain erythrocyte-related information of the subject;
a second test apparatus configured to separately test the test sample solutions corresponding to the respective glycated hemoglobin test modes, so as to obtain a plurality of glycated hemoglobin test results; and
a controller configured to: based on the erythrocyte-related information of the subject, select at least one glycated hemoglobin test result from the plurality of glycated hemoglobin test results, and output the at least one glycated hemoglobin test result.

In this way, after the plurality of glycated hemoglobin test results are obtained, a glycated hemoglobin test result to be output is selected based on the erythrocyte-related information of the subject, so that retesting of the glycated hemoglobin item can be avoided as much as possible.

A fifth aspect of the disclosure relates to a sample testing method, including:
aspirating a blood sample from a container containing a test blood sample of a subject, and performing pretreatment on the aspirated blood sample to obtain a first pretreatment test solution;
testing the first pretreatment test solution to obtain intermediate measurement information of the first pretreatment test solution;
selecting a test mode to be performed from a plurality of different test modes for a same target test item based on the intermediate measurement information of the first pretreatment test solution;
preparing a test sample solution by using a second pretreatment test solution and a test reagent according to the selected test mode to be performed, wherein the second pretreatment test solution is prepared by using the test blood sample of the subject and a pretreatment reagent; and
testing the test sample solution to obtain a test result for the test blood sample for the target test item.

For the advantages and features of the sample testing method provided in the fifth aspect of the disclosure, reference may be made to the sample analyzer provided in the first aspect of the disclosure.

A sixth aspect of the disclosure relates to a sample testing method, including:
preparing test sample solutions respectively corresponding to at least two different test modes based on a test blood sample, wherein each test sample solution is prepared from a pretreatment test solution and a test reagent, and the pretreatment test solution is prepared from the test blood sample and a pretreatment reagent;
testing at least one pretreatment test solution to obtain intermediate measurement information of the at least one pretreatment test solution;
testing the test sample solutions respectively corresponding to the test modes, so as to obtain a plurality of test results for a same target test item; and
based on the intermediate measurement information of the at least one pretreatment test solution, selecting at least one test result from the plurality of test results, and outputting the at least one test result.

For the advantages and features of the sample testing method provided in the sixth aspect of the disclosure, reference may be made to the sample analyzer provided in the second aspect of the disclosure.

A seventh aspect of the disclosure relates to a glycated hemoglobin testing method, including:
when an initial test of a glycated hemoglobin item is performed on a test blood sample of a subject, selecting a test mode to be performed from a plurality of different glycated hemoglobin test modes based on erythrocyte-related information of the subject;
preparing a test sample solution according to the selected test mode to be performed; and
performing a glycated hemoglobin test on the test sample solution.

For the advantages and features of the glycated hemoglobin testing method provided in the seventh aspect of the disclosure, reference may be made to the sample analyzer provided in the third aspect of the disclosure.

An eighth aspect of the disclosure relates to a glycated hemoglobin testing method, including:
under at least two different glycated hemoglobin test modes, preparing test sample solutions corresponding to the respective glycated hemoglobin test modes based on a test blood sample of a subject;
separately testing the test sample solutions corresponding to the respective glycated hemoglobin test modes, so as to obtain a plurality of glycated hemoglobin test results; and
based on erythrocyte-related information of the subject, selecting at least one glycated hemoglobin test result from the plurality of glycated hemoglobin test results, and outputting the at least one glycated hemoglobin test result.

For the advantages and features of the glycated hemoglobin testing method provided in the eighth aspect of the disclosure, reference may be made to the sample analyzer provided in the fourth aspect of the disclosure.

A ninth aspect of the disclosure further provides a sample analyzer, including:
a glycated hemoglobin measurement portion configured to obtain a glycated hemoglobin test result for a blood sample of a subject;
a mode selection portion configured to select a first glycated hemoglobin test mode or a second glycated hemoglobin test mode; and
a controller configured to:
   receive a mode selection of the mode selection portion;
   when the mode selection portion selects the first glycated hemoglobin test mode, control the glycated hemoglobin measurement portion to execute a first measurement process; and
   when the mode selection portion selects the second glycated hemoglobin test mode, control the glycated hemoglobin measurement portion to execute a second measurement process, wherein the second measurement process includes: preparing a first pretreatment test solution by using the blood sample of the subject and a pretreatment reagent, testing the first pretreatment test solution to obtain intermediate measurement information, preparing a test sample solution for the second measurement process by using a second pretreatment test solution and a glycated hemoglobin test reagent based on the intermediate measurement information, and testing the test sample solution for the second measurement process to obtain a glycated hemoglobin test result, wherein the second pretreatment solution is prepared from the blood sample of the subject and a/the pretreatment reagent, and the first measurement process and the second measurement process are at least partially different.

A tenth aspect of the disclosure provides a sample testing method, including:
selecting a first glycated hemoglobin test mode or a second glycated hemoglobin test mode;
when the first glycated hemoglobin test mode is selected, executing a first measurement process; and
when the second glycated hemoglobin test mode is selected, executing a second measurement process, wherein the second measurement process includes: preparing a first pretreatment test solution by using a blood sample of a subject and a pretreatment reagent, testing the first pretreatment test solution to obtain intermediate measurement information, preparing a test sample solution for the second measurement process by using a second pretreatment test solution and a glycated hemoglobin test reagent based on the intermediate measurement information, and testing the test sample solution for the second measurement process to obtain a glycated hemoglobin test result, wherein the second pretreatment solution is prepared from the test blood sample of the subject and a/the pretreatment reagent, and the first measurement process and the second measurement process are at least partially different.

In the solutions proposed in the ninth aspect and the tenth aspect of the disclosure, two test modes are provided for selection, which can improve the accuracy of testing glycated hemoglobin and increase options for users.

### BRIEF DESCRIPTION OF THE DRAWINGS

The disclosure will be more clearly explained below with reference to the embodiments and the accompanying drawings. The above and other advantages will become apparent to those of ordinary skill in the art from the detailed description of the embodiments of the disclosure. The accompanying drawings are only for illustrating preferred embodiments and should not be considered as limiting the disclosure. The same or similar reference numerals represent the same components throughout the accompanying drawings. In the accompanying drawings:
FIG. 1 is a schematic block diagram of a sample analyzer according to a first implementation of the disclosure;
FIG. 2 is a schematic structure diagram of the sample analyzer in FIG. 1;
FIG. 3 is a schematic structure diagram of a first test apparatus according to an embodiment of the disclosure;
FIG. 4 is an image of a first pretreatment test solution according to an embodiment of the disclosure;
FIG. 5 is a schematic flowchart of a glycated hemoglobin test according to an embodiment of the disclosure;
FIG. 6 is a graph showing a relationship between an absorbance difference and an estimated hemoglobin concentration according to an embodiment of the disclosure;
FIG. 7 is another schematic flowchart of a glycated hemoglobin test according to an embodiment of the disclosure;
FIG. 8 is a schematic block diagram of a sample analyzer according to a second implementation of the disclosure;
FIG. 9 is a schematic block diagram of a sample analyzer according to a third implementation of the disclosure;
FIG. 10 is a schematic block diagram of a sample analyzer according to a fourth implementation of the disclosure;
FIG. 11 is a schematic flowchart of a sample testing method according to a fifth implementation of the disclosure;
FIG. 12 is a schematic flowchart of a sample testing method according to a sixth implementation of the disclosure;
FIG. 13 is a schematic flowchart of a glycated hemoglobin testing method according to a seventh implementation of the disclosure;
FIG. 14 is a schematic flowchart of a glycated hemoglobin testing method according to an eighth implementation of the disclosure;
FIG. 15 is a schematic block diagram of a glycated hemoglobin analyzer according to a ninth implementation of the disclosure; and
FIG. 16 is a schematic flowchart of a glycated hemoglobin testing method according to a tenth implementation of the disclosure.

### DESCRIPTION OF THE PREFERRED EMBODIMENTS

The embodiments of the disclosure will be described below clearly and completely in conjunction with the accompanying drawings. Obviously, the embodiments described are merely some, rather than all, of the embodiments of the disclosure. Based on the embodiments of the disclosure, all the other embodiments that would have been obtained by those of ordinary skill in the art without any creative efforts shall fall within the scope of protection of the disclosure.

It should be noted that the term "first/second/third" in the embodiments of the disclosure is only used to distinguish similar objects, and does not represent specific order for the objects. It may be understood that "first/second/third" may be interchanged for specific order or chronological order when allowed.

It can be understood by those skilled in the art that unless otherwise defined, all terms (including technical terms and scientific terms) used herein have the same meanings as those generally understood by those of ordinary skill in the art to which the disclosure belongs.

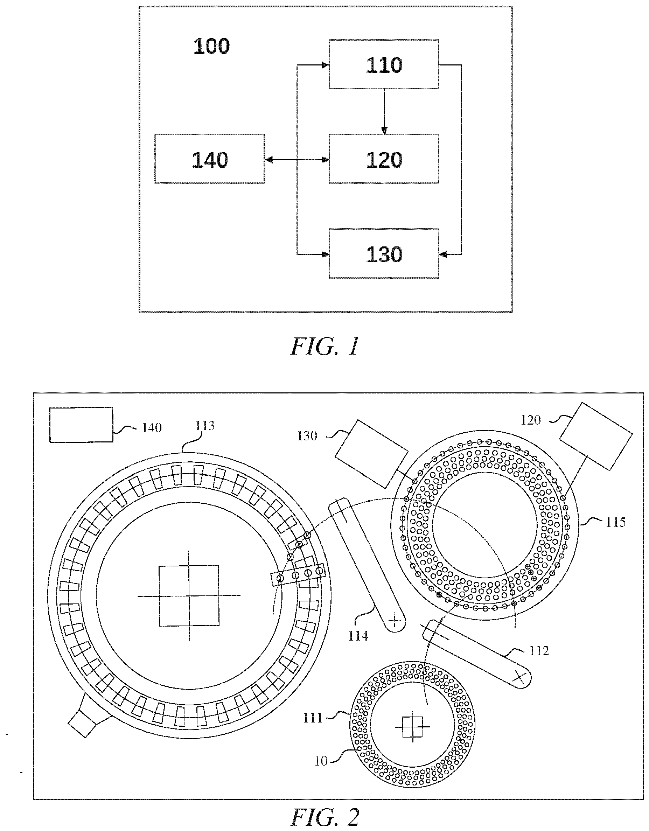

FIG. 1 is a schematic block diagram of a sample analyzer 100 according to a first implementation of the disclosure. The sample analyzer 100 includes a sample preparation apparatus 110, a first test apparatus 120, a second test apparatus 130, and a controller 140.

The sample preparation apparatus 110 is configured to aspirate a blood sample from a container 10 containing a test blood sample, such as a whole blood sample of a subject, and prepare a first pretreatment test solution by using the aspirated blood sample and a pretreatment reagent.

The first test apparatus 120 is configured to test the first pretreatment test solution prepared by the sample preparation apparatus 110, so as to obtain intermediate measurement information of the first pretreatment test solution.

The controller 140 is configured to: select a test mode to be performed from a plurality of different test modes based on the intermediate measurement information obtained by the first test apparatus 120, and control the sample preparation apparatus 120 to prepare a test sample solution by using a second pretreatment test solution and a test reagent according to the selected test mode to be performed, wherein the second pretreatment test solution is prepared by the sample preparation apparatus 110 using the test blood sample of the subject and a/the pretreatment reagent.

The second test apparatus 130 is configured to test the test sample solution, to obtain a test result for the test blood sample for a target test item.

Unlike the case in the prior art that, for an abnormal blood sample, it is required to prepare a test sample solution twice and perform a target item test twice, in this embodiment of the disclosure, before a test sample solution finally used in the second test apparatus 130 is prepared, an intermediate test solution, that is, a pretreatment test solution, is tested to obtain intermediate measurement information, and a test mode, particularly, a preparation condition, for preparing the test sample solution is selected based on the intermediate measurement information, so as to prepare the test sample solution suitable for the current test blood sample. An accurate test result can be obtained through one test, and sample retesting can be reduced, thereby improving the overall testing efficiency of the sample analyzer.

In a specific embodiment, as shown in FIG. 2, the sample analyzer 100 is configured as a biochemical instrument. In the embodiment shown in FIG. 2, the sample preparation apparatus 110 includes a sample holding component 111, a sample dispensing mechanism 112, a reagent holding component 113, a reagent dispensing mechanism 114, and a reaction component 115.

The sample holding component 111 is configured to hold blood samples. For example, the sample holding component 111 may be configured as a sample disc, the sample disc includes a plurality of sample positions for placing containers 10, and the sample disc can be rotated to dispatch a container containing a blood sample to a corresponding position, for example, to a position in which the sample dispensing mechanism 112 aspirates a blood sample. The sample dispensing mechanism 112 is configured to aspirate a blood sample from a container 10 and discharge the blood sample into a reaction cup, which is to be subjected to sample addition. For example, the sample dispensing mechanism 112 may include a sample needle, and the sample needle may perform a two-dimensional or three-dimensional movement in space by using a two-dimensional or three-dimensional driving mechanism, so that the sample needle can move to a position for aspirating a blood sample, move to a reaction cup to be subjected to sample addition, and discharge the aspirated blood sample into the reaction cup.

The reagent holding component 113 is configured to hold reagents including a pretreatment reagent and a test reagent. In some embodiments, the reagent holding component 113 may be configured as a reagent disc having a circular disc structure and provided with a plurality of positions for holding reagent containers. The reagent holding component 113 can rotate and drive a reagent container held therein to rotate to a specific position, for example, a position that allows the reagent dispensing mechanism 114 to aspirate a reagent. There may be one or more reagent holding components 113. The reagent dispensing mechanism 114 is configured to aspirate a reagent and discharge the reagent into a reaction cup, which is to be subjected to reagent addition. For example, the reagent dispensing mechanism 114 may include a reagent needle, and the reagent needle may perform a two-dimensional or three-dimensional movement in space by using a two-dimensional or three-dimensional driving mechanism, so that the reagent needle can move to a position for aspirating a reagent, move to a reaction cup to be subjected to reagent addition, and discharge the aspirated reagent into the reaction cup.

The reaction component 115 has at least one placement position, which is configured to place a reaction cup and incubate a test solution, for example, the pretreatment test solution and the test sample solution, in the reaction cup. For example, the reaction component 115 may be configured as a reaction disc having a circular disc structure and provided with one or more placement positions for placing reaction cups. The reaction disc can rotate and drive a reaction cup in a placement position to move, so as to dispatch the reaction cup in the reaction disc and incubate the test solution in the reaction cup.

The first test apparatus 120 is configured to test the first pretreatment test solution in the reaction component 115 that is obtained through mixing of the blood sample with the pretreatment reagent, so as to obtain the intermediate measurement information of the first pretreatment test solution. For example, the first test apparatus 120 is arranged outside the reaction component 115, and the reaction component 115 rotates to drive the container containing the first pretreatment test solution to move to the first test apparatus 130 for testing.

The second test apparatus 130 is configured to test the test sample solution that has been incubated in the reaction component 115, so as to obtain a test result for the target test item. For example, the second test apparatus 140 is also arranged outside the reaction component 115, and the reaction component 115 rotates to drive the container containing the test sample solution to move to the second test apparatus 130 for the target item test. In some embodiments, the second test apparatus 130 is configured as a luminescence detection apparatus.

Further, in the embodiment shown in FIG. 2, the sample preparation apparatus 110 may further include a test solution transferring part (not shown) configured to transfer the pretreatment test solution to a corresponding reaction cup. In some embodiments, the sample dispensing mechanism 112 may be used as the test solution transferring part.

In some embodiments, the pretreatment reagent for preparing the first pretreatment test solution may include a hemolytic agent for lysing erythrocytes in the test blood sample to release hemoglobin, and the intermediate measurement information includes a measurement value related to a hemoglobin concentration of the first pretreatment test solution, for example, the intermediate measurement information includes the hemoglobin concentration of the first pretreatment test solution. That is, herein, the test mode to be performed is selected based on the measurement value related to the hemoglobin concentration of the first pretreatment test solution.

Alternatively or additionally, the pretreatment reagent for preparing the first pretreatment test solution may include a hemolytic agent for lysing erythrocytes in the test blood sample to release hemoglobin, and the intermediate measurement information includes a measurement value related to an interferent in the first pretreatment test solution that interferes with the target test item. The interferent is, for example, a lipid interferent, and the measurement value related to the interferent is, for example, a lipemia concentration or a bilirubin concentration. That is, herein, the test mode to be performed is selected based on the measurement value related to the interferent in the first pretreatment test solution.

In some other alternative embodiments, the pretreatment reagent for preparing the first pretreatment test solution may include a diluent for diluting the test blood sample to measure a hematocrit, and the intermediate measurement information includes a measurement value related to a hematocrit of the first pretreatment test solution, for example, the intermediate measurement information includes the hematocrit of the first pretreatment test solution. That is, herein, the test mode to be performed is selected based on the measurement value related to the hematocrit of the first pretreatment test solution.

In some embodiments, the target test item may be a glycated hemoglobin test item, and the second test apparatus may be further configured to measure glycated hemoglobin in the test blood sample.

Glycated hemoglobin, also known as glycosylated hemoglobin, is the product of non-enzymatic catalytic reaction of glucose and other sugars with the amino group of hemoglobin, and its content is positively correlated with a blood glucose concentration in the blood. Since a protein glycation reaction is an irreversible reaction, and a lifespan of an erythrocyte in the blood circulation is 120 days, glycated hemoglobin can reflect an average blood glucose concentration in the past three months and is widely used in clinical diagnosis and treatment monitoring as an indicator of blood glucose control.

In clinical testing, the ratio or percentage of glycated hemoglobin in hemoglobin is usually used as a reporting unit, mainly including IFCC (mmol/mol), NGSP (%) in the United States, JDS/JSCC (%) in Japan, and MonoS (%) in Sweden.

Existing measurement techniques for glycated hemoglobin include immunoaffinity chromatography, ion exchange chromatography, capillary electrophoresis, turbidimetric inhibition immunoassay, and enzymatic assay. In these measurement methods, retesting may be caused by factors such as an interference of turbid samples or a test result exceeding an effective measurement range. The effective measurement range is a variation range of a concentration of an analyte in which test results obtained by a method have precision and accuracy meeting requirements and are linear. Therefore, using the technical solution proposed in the disclosure can be advantageously used to reduce retesting of the glycated hemoglobin test item.

Taking turbidimetric inhibition immunoassay and enzymatic assay for measuring glycated hemoglobin as an example, in some embodiments, the controller 140 may be further configured to:
control the sample preparation apparatus 110 to prepare a first test sample solution by using the second pretreatment test solution and a first test reagent for the glycated hemoglobin test item according to the selected test mode to be performed, wherein the first test reagent includes an enzyme reagent (such as a proteolytic enzyme, used for acting on glycated hemoglobin in the second pretreatment test solution to produce glycosylated peptides or glycosylated amino acids) or an immunological reagent (such as a glycated hemoglobin-specific antibody) for measuring a hemoglobin content;
control the second test apparatus 130 to test the first test sample solution, so as to obtain a hemoglobin content of the test blood sample;
control the sample preparation apparatus 110 to add a second test reagent for the glycated hemoglobin test item to the first test sample solution, so as to prepare a second test sample solution, wherein the second test reagent includes an enzyme reagent (such as a specific oxidase, used to react with the glycosylated peptide or glycosylated amino acid in the first test sample solution to produce detectable products, such as hydrogen peroxide) or an immunological reagent (such as multi-cluster single-antigens carrying a number of glycated hemoglobin antigenic determinants) for measuring glycated hemoglobin;
control the second test apparatus 130 to test the second test sample solution, so as to obtain a glycated hemoglobin content of the test blood sample; and
calculate a ratio of the glycated hemoglobin content to the hemoglobin content as a test result for the glycated hemoglobin test item.

In some other embodiments, the second test apparatus 130 may be configured as a test apparatus based on ion exchange chromatography. In this case, the test reagent includes an equilibration buffer solution and an elution buffer solution.

In still some other embodiments, the second test apparatus 130 may be configured as a test apparatus that works based on affinity chromatography. In this case, the test reagent includes the aminophenylboronic acid-agarose gel, the asparagine buffer solution, the sorbitol buffer solution (eluent), and the like.

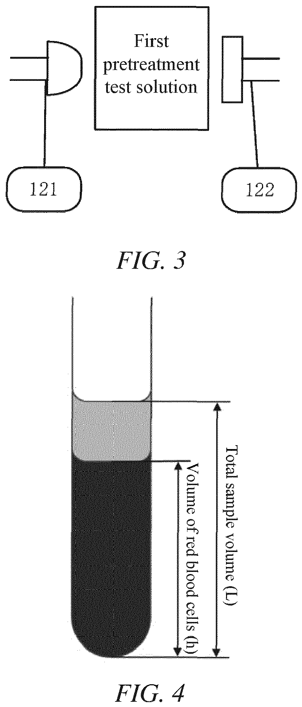

In some embodiments, as shown in FIG. 3, the first test apparatus 120 may be further configured to irradiate the first pretreatment test solution with a light of at least one wavelength, and obtain optical signals generated after the first pretreatment test solution is irradiated with the light, so as to obtain the intermediate measurement information based on the optical signals. In the embodiment shown in FIG. 3, the first test apparatus 120 includes a light source 121 and a light detector 122. The light source 121 is configured to irradiate the first pretreatment test solution with a light of at least one wavelength, and the light detector 122 is configured to detect optical signals (transmitted light and/or scattered light) emitted by the light source 121 and passing through the first pretreatment test solution, so as to obtain the intermediate measurement information based on the optical signals. In the embodiment shown in FIG. 3, the pretreatment reagent particularly includes a hemolytic agent for lysing erythrocytes in the test blood sample, particularly, the whole blood sample, to release hemoglobin, and the intermediate measurement information includes a measurement value related to a hemoglobin concentration of the first pretreatment test solution (the intermediate measurement information preferably includes the hemoglobin concentration of the first pretreatment test solution), and/or the intermediate measurement information includes a measurement value related to an interferent in the first pretreatment test solution that interferes with the target test item.

In some other embodiments, the pretreatment reagent includes a hemolytic agent for lysing erythrocytes in the test blood sample to release hemoglobin. Herein, the first test apparatus 120 is configured as a camera, and the camera is configured to image the first pretreatment test solution to obtain an image of the first pretreatment test solution. The measurement value related to the hemoglobin concentration of the first pretreatment test solution may be obtained based on the image, for example, the hemoglobin concentration of the first pretreatment test solution is estimated based on depth of red in the image.

Glycated hemoglobin is the product of continuous and irreversible non-enzymatic glycosylation reaction of hemoglobin with glucose in erythrocytes. For a same test specimen, a glycated hemoglobin concentration of the specimen has a linear relationship with a hemoglobin concentration (HGB) of the specimen. A large number of experiments have proved that a hemoglobin concentration of a blood sample also has a linear relationship with a hematocrit (i.e., "erythrocyte volume /total sample volume" (HCT)) of the blood sample after centrifugation or natural sedimentation. Therefore, the test mode may be selected based on the HCT value of the blood sample after centrifugation or natural sedimentation. That is, in still some other embodiments, the sample preparation apparatus 110 is configured to perform centrifugation or natural sedimentation on the test blood sample, so as to obtain the pretreatment test solution. The first test apparatus 120 is configured as a camera and is configured to image the first pretreatment test solution after the centrifugation or natural sedimentation, so as to obtain an image of the first pretreatment test solution. The measurement value related to the hemoglobin concentration of the first pretreatment test solution may be obtained based on the image. For example, as shown in FIG. 4, based on a proportion of erythrocytes at the bottom to plasma in the upper layer after the centrifugation or the natural sedimentation or based on a proportion of erythrocyte volume h of the first pretreatment test solution after the centrifugation or the natural sedimentation to total sample volume L, the hematocrit HCT (=h/L) of the first pretreatment test solution may be obtained, and then a glycated hemoglobin concentration of the first pretreatment test solution may be estimated. Preferably, it takes at least half an hour for the natural sedimentation to obtain a better image effect.

In some other alternative embodiments, the first test apparatus 120 is further configured to allow the first pretreatment test solution to pass through, so as to obtain pulse measurement signals, such that the intermediate measurement information is obtained based on the pulse measurement signals. In this case, the first test apparatus 120 is configured, for example, as a resistance measuring apparatus. Herein, the pretreatment reagent preferably includes a diluent for diluting the test blood sample to measure a hematocrit, and the intermediate measurement information includes a hematocrit of the first pretreatment test solution.

In some embodiments, the first test apparatus 120 and the second test apparatus 130 may be a same test apparatus, to further reduce costs. For example, the first test apparatus 120 and the second test apparatus 130 are the optical detection apparatus shown in FIG. 3.

In the embodiments of the disclosure, after the test mode to be performed is selected based on the intermediate measurement information obtained by testing the first pretreatment test solution, the test sample solution may be prepared in the following manners.

In a first manner of preparing the test sample solution, the controller 140 may be further configured to: when the sample preparation apparatus 110 is controlled to prepare the test sample solution by using the second pretreatment test solution and the test reagent according to the selected test mode to be performed, control the sample preparation apparatus to prepare the test sample solution by using the test reagent and the first pretreatment test solution, wherein the first pretreatment test solution is used as the second pretreatment test solution. That is, the first pretreatment test solution and the second pretreatment test solution are a same pretreatment test solution, for example, the sample preparation apparatus 110 mixes a part of the first pretreatment test solution with the test reagent to obtain the test sample solution. Therefore, the overall testing efficiency of the sample analyzer can be improved, and the overall testing cost can also be reduced.

Taking the sample analyzer shown in FIG. 2 as an example to describe the first manner of preparing the test sample solution. The controller 140 controls the reagent dispensing mechanism 114 to add a pretreatment reagent, such as a hemolytic agent in the reagent holding component 113 to a first reaction cup in the reaction component 115. Then, the controller 140 controls the sample dispensing mechanism 112 to aspirate a part of a test blood sample, particularly a whole blood sample from a container 10, and to add the part of the test blood sample to the first reaction cup to which the pretreatment reagent has been added, so as to obtain a first pretreatment test solution. The controller 140 controls the first test apparatus 130 to test the first pretreatment test solution in the first reaction cup, so as to obtain intermediate measurement information, such as a hemoglobin concentration of the first pretreatment test solution. The controller 140 selects a test mode to be performed based on the intermediate measurement information of the first pretreatment test solution. Then, the first pretreatment test solution is incubated in the reaction component 115 for a period of time. Next, the controller 140 controls the reagent dispensing mechanism 114 to add a test reagent in the reagent holding component 113 to a second reaction cup located in the reaction component 115, and controls the test solution transferring part to transfer a part of the first pretreatment test solution in the first reaction cup to the second reaction cup to which the test reagent has been added, so as to prepare a test sample solution. Then, the controller 140 controls the second test apparatus 130 to test the test sample solution in the second reaction cup.

In a second manner of preparing the test sample solution, the controller 140 may be further configured to: when the sample preparation apparatus 110 is controlled to prepare the test sample solution by using the second pretreatment test solution and the test reagent according to the selected test mode to be performed, control the sample preparation apparatus 110 to aspirate a blood sample of the subject again, prepare the second pretreatment test solution based on the blood sample aspirated again, and prepare the test sample solution based on the second pretreatment test solution prepared and the test reagent. That is, the sample preparation apparatus 110 does not use the first pretreatment test solution, but uses the second pretreatment test solution prepared by using the blood sample aspirated again, to prepare the test sample solution.

Also, taking the sample analyzer shown in FIG. 2 as an example to describe the second manner of preparing the test sample solution. The controller 140 controls the reagent dispensing mechanism 114 to add a pretreatment reagent, such as a hemolytic agent in the reagent holding component 113 to a first reaction cup in the reaction component 115. Then, the controller 140 controls the sample dispensing mechanism 112 to aspirate a part of a test blood sample, particularly a whole blood sample from a container 10, and to add the part of the test blood sample to the first reaction cup to which the pretreatment reagent has been added, so as to obtain a first pretreatment test solution. The controller 140 controls the first test apparatus 130 to test the first pretreatment test solution in the first reaction cup, so as to obtain intermediate measurement information, such as a hemoglobin concentration of the first pretreatment test solution. The controller 140 selects a test mode to be performed based on the intermediate measurement information of the first pretreatment test solution. Then, the controller 140 controls, according to the test mode to be performed, the reagent dispensing mechanism 114 to add the pretreatment reagent, such as the hemolytic agent in the reagent holding component 113 to a second reaction cup located in the reaction component 115. Then the controller 140 controls the sample dispensing mechanism 112 to aspirate another part of the test blood sample from the container 10 again or aspirate a part of the test blood sample from another container containing a test blood sample of the same subject, and to add the part of the test blood sample to the second reaction cup to which the pretreatment reagent has been added, so as to obtain a second pretreatment test solution. The second pretreatment test solution is incubated in the reaction component 115 for a period of time. Next, the controller 140 controls the reagent dispensing mechanism 114 to add a test reagent in the reagent holding component 113 to a third reaction cup located in the reaction component 115, and controls the test solution transferring part to transfer a part of the second pretreatment test solution in the second reaction cup to the third reaction cup to which the test reagent has been added, so as to prepare a test sample solution. Then, the controller 140 controls the second test apparatus 130 to test the test sample solution in the third reaction cup.

In a third manner of preparing the test sample solution, the sample preparation apparatus 110 may be further configured to prepare a plurality of pretreatment test solutions by using the test blood sample and the pretreatment reagent, wherein the plurality of pretreatment test solutions are prepared differently from each other in terms of at least one of component, formula and dosage of the pretreatment reagent, and/or the plurality of pretreatment test solutions are prepared differently from each other in terms of dosage of the test blood sample. The first test apparatus 120 may be further configured to test at least one first pretreatment test solution in the plurality of pretreatment test solutions, so as to obtain the intermediate measurement information of the first pretreatment test solution. In addition, in this case, the controller 140 may be further configured to: when the sample preparation apparatus 110 is controlled to prepare the test sample solution by using the second pretreatment test solution and the test reagent according to the selected test mode to be performed, control the sample preparation apparatus 110 to select one pretreatment test solution from the plurality of pretreatment test solutions as the second pretreatment test solution according to the selected test mode to be performed, and prepare the test sample solution by using the selected pretreatment test solution and the test reagent.

Also, taking the sample analyzer shown in FIG. 2 as an example to describe the third manner of preparing the test sample solution. The controller 140 controls the reagent dispensing mechanism 114 to separately add a pretreatment reagent, such as a hemolytic agent in the reagent holding component 113 to a first reaction cup, a second reaction cup, and a third reaction cup in the reaction component 115. Then, the controller 140 controls the sample dispensing mechanism 112 to separately add a test blood sample in a container 10 to the first reaction cup, the second reaction cup, and the third reaction cup to which the pretreatment reagent has been added, so as to obtain a plurality of pretreatment test solutions. The controller 140 controls the first test apparatus 130 to test the first pretreatment test solution in the first reaction cup, so as to obtain intermediate measurement information, such as a hemoglobin concentration of the first pretreatment test solution. The controller 140 selects a test mode to be performed based on the intermediate measurement information of the first pretreatment test solution. Then, the controller 140 controls, according to the test mode to be performed, the reagent dispensing mechanism 114 to add a test reagent in the reagent holding component 113 to a fourth reaction cup located in the reaction component 115, and controls the test solution transferring part to transfer a part of the second pretreatment test solution in the second reaction cup to the fourth reaction cup to which the test reagent has been added, so as to prepare a test sample solution. Then, the controller 140 controls the second test apparatus 130 to test the test sample solution in the fourth reaction cup.

In some embodiments, the plurality of different test modes include at least two test modes, and effective test ranges of the at least two test modes are at least partially non-overlapping. Herein, the intermediate measurement information of the first pretreatment test solution can be used to estimate a test result of the test blood sample for the target test item, for example, the hemoglobin concentration or the hematocrit of the first pretreatment test solution can be used to estimate a glycated hemoglobin concentration of the test blood sample, so that a test mode suitable for the effective test range of the test blood sample can be selected based on the intermediate measurement information of the first pretreatment test solution, thereby preventing the test result from exceeding the effective test range, and thereby implementing testing for the target item through one test.

In the embodiments of the disclosure, the effective test range is a variation range of a concentration of an analyte in which test results obtained by a method have precision and accuracy meeting requirements and are linear.

In some embodiments, the plurality of different test modes may differ in at least one of the following aspects:
a dosage of at least one of the test blood sample, the pretreatment reagent and the test reagent used to prepare the test sample solution, or in the test sample solution, a proportion of at least one of the test blood sample, the pretreatment reagent and the test reagent used to prepare the test sample solution;
a component, a formula or a dosage of the pretreatment reagent used to prepare the test sample solution; and
a type or a dosage of the test reagent used to prepare the test sample solution.

For example, when it is determined that the glycated hemoglobin concentration of the test blood sample is relatively low, a test mode in which a sample volume is increased is enabled to increase the glycated hemoglobin concentration in the reaction system, and to reduce the impact of a non-specific background reaction on measurement of the glycated hemoglobin concentration, thereby solving the problem that results are inaccurate during testing of low-concentration glycated hemoglobin, and achieving the effect of obtaining an accurate glycated hemoglobin test result through one test.

Alternatively or additionally, the second test apparatus is further configured to test the test sample solution according to the selected test mode to be performed, so as to obtain the test result for the test blood sample for the target test item. Herein, the plurality of different test modes may differ in at least one of the following aspects:
a wavelength of light for testing the test sample solution for the target test item; and
a methodology for testing the test sample solution for the target test item.

In some embodiments, the pretreatment reagent for preparing the first pretreatment test solution includes a hemolytic agent for lysing erythrocytes in the test blood sample to release hemoglobin, and the intermediate measurement information includes a measurement value related to a hemoglobin concentration of the first pretreatment test solution; or the pretreatment reagent for preparing the first pretreatment test solution includes a diluent for diluting the test blood sample to measure a hematocrit, and the intermediate measurement information includes a measurement value related to a hematocrit of the first pretreatment test solution. The target test item is a glycated hemoglobin test item, and the second test apparatus 130 is further configured to measure glycated hemoglobin in the test blood sample. In this case, the controller 140 is further configured to:
when the measurement value related to the hemoglobin concentration and/or the hematocrit of the first pretreatment test solution are/is within a preset range, select a first test mode as the test mode to be performed; and
when the measurement value related to the hemoglobin concentration and/or the hematocrit of the first pretreatment test solution are/is greater than an upper limit of the preset range, select a second test mode as the test mode to be performed, wherein in the test sample solution, a proportion of the test blood sample or the second pretreatment test solution used to prepare the test sample solution under the first test mode is greater than a proportion of the test blood sample or the second pretreatment test solution used to prepare the test sample solution under the second test mode; and/or when the measurement value related to the hemoglobin concentration and/or the hematocrit of the first pretreatment test solution are/is less than a lower limit of the preset range, select a third test mode as the test mode to be performed, wherein in the test sample solution, the proportion of the test blood sample or the second pretreatment test solution used to prepare the test sample solution under the first test mode is less than a proportion of the test blood sample or the second pretreatment test solution used to prepare the test sample solution under the third test mode.

Herein, the dosage of the test blood sample or the second pretreatment test solution used for preparing the test sample solution may be increased and/or the volume of the test sample solution may be reduced, so as to increase the proportion of the test blood sample or the second pretreatment test solution used to prepare the test sample solution in the test sample solution. Likewise, the dosage of the test blood sample or the second pretreatment test solution used for preparing the test sample solution may be reduced and/or the volume of the test sample solution may be increased, so as to reduce the proportion of the test blood sample or the second pretreatment test solution used to prepare the test sample solution in the test sample solution.

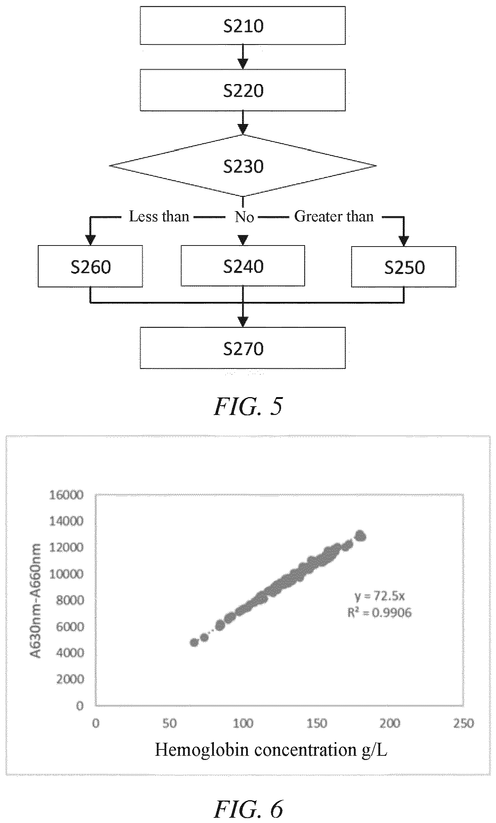

FIG. 5 shows a specific process of a glycated hemoglobin test. In step S210, the sample preparation apparatus 110 mixes a test blood sample with a hemolytic agent to obtain a first pretreatment test solution. In step S220, the first test apparatus 120 detects optical signals in a process of the test blood sample reacting with the hemolytic agent, to obtain an estimated hemoglobin concentration of the first pretreatment test solution based on the optical signals. In step S230, the controller 140 determines whether the estimated hemoglobin concentration exceeds a preset range. When the estimated hemoglobin concentration does not exceed the preset range, step S240 is performed: the sample preparation apparatus 110 prepares the test sample solution according to the first test mode. When the estimated hemoglobin concentration is greater than an upper limit of the preset range, step S250 is performed: the sample preparation apparatus 110 prepares the test sample solution according to the second test mode. When the estimated hemoglobin concentration is less than a lower limit of the preset range, step S260 is performed: the sample preparation apparatus 110 prepares the test sample solution according to the third test mode. In step S270, the second test apparatus 130 performs a glycated hemoglobin test on the test sample solution.

An example of the disclosure is described by taking measuring glycated hemoglobin based on enzymatic assay as an example. First, a hemoglobin measurement range of the enzymatic assay is determined to be 70 g/L-400 g/L through a plurality of samples with gradient hemoglobin concentrations, wherein an upper limit of measurement is 400 g/L, and a lower limit of measurement is 70 g/L. A specific manner of determining the hemoglobin measurement range is, for example: using a sample of a upper limit of measurement and a sample of a lower limit of measurement to configure equal-proportional gradient samples, and testing a result for each sample; performing linear regression on the results for all the samples, and determining, through calculation, whether a regression deviation of each concentration point is less than a total error of the glycated hemoglobin test item; if the regression deviation is less than the total error, using the upper limit of measurement and the lower limit of measurement as a measurement range of the sample analyzer; and if the regression deviation is greater than the total error, narrowing an interval between the upper limit of measurement and the lower limit of measurement, and repeating the experiment until error requirements are met. Due to narrow distribution of a content of glycated hemoglobin in hemoglobin (4%-14%) in the population, the hemoglobin measurement range may also be used to represent a glycated hemoglobin measurement range. In a specific testing process, a test whole blood sample is first lysed with a hemolytic agent (that is, the pretreatment reagent) according to a certain proportion, such as 1 : 23, so as to obtain a first pretreatment test solution, wherein the hemolytic agent contains a nitrite and other components that can oxidize hemoglobin into methemoglobin. The first pretreatment test solution is used for subsequent preparation of a test sample solution, after the hemolytic agent is mixed with the test whole blood sample, the hemoglobin is oxidized into methemoglobin, and an estimated hemoglobin concentration of the first pretreatment test solution may be obtained by measuring absorbance of the first pretreatment test solution at a wavelength of 630 nm; or an estimated hemoglobin concentration may be obtained through measurement of a difference A between the absorbance of the first pretreatment test solution at a dominant wavelength of 630 nm and the absorbance of the first pretreatment test solution at a subwavelength of 660 nm (used to reduce noise and interference from lactone, and the like), wherein the difference A of absorbance has a linear relationship with the estimated hemoglobin concentration g/L, which is specifically as follows: Hemoglobin concentration g/L=A_((630 nm-660 nm))/72.5, as shown in FIG. 6. Then, the estimated hemoglobin concentration is compared with preset upper and lower limits of hemoglobin measurement, and a dosage of the first pretreatment test solution involved in the subsequent preparation of the test sample solution is determined. When the estimated hemoglobin concentration is between the preset upper and lower limits of hemoglobin measurement, the first test mode is selected, and 5 µl of the first pretreatment test solution is used to prepare the test sample solution; when the estimated hemoglobin concentration is greater than the preset upper limit of hemoglobin measurement, the second test mode is selected, and 3 µl of the first pretreatment test solution is used to prepare the test sample solution; and when the estimated hemoglobin concentration is less than the preset lower limit of hemoglobin measurement, the third test mode is selected, and 8 µl of the first pretreatment test solution is used to prepare the test sample solution. Herein, in the first test mode, the second test mode, and the third test mode, the dosage of the test reagent used to prepare the test sample solution is the same. After the dosage of the first pretreatment test solution is determined, the determined first pretreatment test solution is mixed with a first test reagent containing a proteolytic enzyme to obtain a first test sample solution, the proteolytic enzyme acts on glycated hemoglobin in the test solution to produce a glycosylated peptide or glycosylated amino acid, absorbance of the first test sample solution at a wavelength of 480 nm is measured at the same time, and a hemoglobin concentration of the test blood sample is obtained based on the absorbance and a calibration curve. Subsequently, a second test reagent containing a specific oxidase is added to the first test sample solution to obtain a second test sample solution, and the specific oxidase reacts with the glycosylated peptide or glycosylated amino acid to produce detectable products (such as hydrogen peroxide), a chromogenic reaction occurs between the hydrogen peroxide and 10-(carboxymethylaminocarbonyl)-3,7-bis(dimethylamino)phenothiazine sodium (color developing agent), and a glycated hemoglobin concentration is obtained by measuring the absorbance. Finally, a glycated hemoglobin test result is obtained based on the hemoglobin concentration and the glycated hemoglobin concentration.

Another example of the disclosure is described by taking measuring glycated hemoglobin based on turbidimetric inhibition immunoassay as an example. Likewise, a hemoglobin measurement range of the turbidimetric inhibition immunoassay is first determined to be 40 g/L-410 g/L through a plurality of samples with gradient hemoglobin concentrations, wherein an upper limit of measurement is 410 g/L, and a lower limit of measurement is 40 g/L. Due to narrow distribution of a content of glycated hemoglobin in hemoglobin (4%-14%) in the population, the hemoglobin measurement range may also be used to represent a glycated hemoglobin measurement range. In a specific testing process, a test whole blood sample is lysed with a hemolytic agent (that is, the pretreatment reagent) according to a certain proportion, such as 1:50, so as to obtain a first pretreatment test solution, wherein the hemolytic agent contains a nitrite and other components that can oxidize hemoglobin into methemoglobin. The first pretreatment test solution is used for subsequent preparation of a test sample solution. After the hemolytic agent is mixed with the test whole blood sample, the hemoglobin is oxidized into methemoglobin, and an estimated hemoglobin concentration of the first pretreatment test solution may be obtained by measuring absorbance of the first pretreatment test solution at a wavelength of 540 nm or 570 nm. Then, the estimated hemoglobin concentration is compared with preset upper and lower limits of hemoglobin measurement, and a dosage of the first pretreatment test solution involved in the subsequent preparation of the test sample solution is determined. When the estimated hemoglobin concentration is between the preset upper and lower limits of hemoglobin measurement, the first test mode is selected, and 6 µl of the first pretreatment test solution is used to prepare the test sample solution; and when the estimated hemoglobin concentration is greater than the preset upper limit of hemoglobin measurement, the second test mode is selected, and 4 µl of the first pretreatment test solution is used to prepare the test sample solution. Herein, in the first test mode and the second test mode, a dosage of the test reagent used to prepare the test sample solution is the same. After the dosage of the first pretreatment test solution is determined, the determined first pretreatment test solution is mixed with a first test reagent containing a glycated hemoglobin-specific antibody to obtain a first test sample solution, and at the same time, optical measurement is performed on the first test sample solution to obtain a hemoglobin concentration of the test blood sample. Subsequently, a second test reagent that contains multi-cluster single-antigens carrying a number of glycated hemoglobin antigenic determinants is added to the first test sample solution to obtain a second test sample solution. The multi-cluster single-antigens react with excessive antibodies in the first test sample solution to form insoluble complexes. If a glycated hemoglobin concentration of the second test sample solution is increased, the formation of immune complexes is reduced, and the turbidity is reduced, thereby measuring a glycated hemoglobin concentration of the second test sample solution. Finally, a glycated hemoglobin test result is obtained based on the hemoglobin concentration and the glycated hemoglobin concentration.

Another example of the disclosure is described by taking measuring glycated hemoglobin based on enzymatic assay as an example. First, a hemoglobin measurement range of the enzymatic assay is determined to be 70 g/L-400 g/L through a plurality of samples with gradient hemoglobin concentrations, wherein an upper limit of measurement is 400 g/L, and a lower limit of measurement is 70 g/L. In a specific testing process, a test whole blood sample is lysed with a hemolytic agent (that is, the pretreatment reagent) according to a certain proportion, such as 1:23, so as to obtain a first pretreatment test solution, wherein the hemolytic agent contains a nitrite and other components that can oxidize hemoglobin into methemoglobin. The first pretreatment test solution is used for subsequent preparation of a test sample solution. After the hemolytic agent is mixed with the test whole blood sample, the hemoglobin is oxidized into methemoglobin, and an estimated hemoglobin concentration of the first pretreatment test solution may be obtained by measuring absorbance of the first pretreatment test solution at a wavelength of 630 nm. Then, the estimated hemoglobin concentration is compared with preset upper and lower limits of hemoglobin measurement, and a dosage of the first pretreatment test solution involved in the subsequent preparation of the test sample solution is determined. When the estimated hemoglobin concentration is between the preset upper and lower limits of hemoglobin measurement, the first test mode is selected, and 5 µl of the first pretreatment test solution, 90 µl of a first test reagent containing a proteolytic enzyme, and 30 µl of a second test reagent containing a specific oxidase are used to prepare the test sample solution; when the estimated hemoglobin concentration is greater than the preset upper limit of hemoglobin measurement, the second test mode is selected, and 5 µl of the first pretreatment test solution, 120 µl of the first test reagent containing the proteolytic enzyme, and 40 µl of the second test reagent containing the specific oxidase are used to prepare the test sample solution; and when the estimated hemoglobin concentration is less than the preset lower limit of hemoglobin measurement, the third test mode is selected, and 5 µl of the first pretreatment test solution, 60 µl of the first test reagent containing the proteolytic enzyme, and 20 µl of the second test reagent containing the specific oxidase are used to prepare the test sample solution. Herein, a relative adjustment of the first pretreatment test solution is implemented by adjusting the dosages of the test reagents.

In some other examples, a proportion of the dosage of the first pretreatment test solution to the dosage of the test reagent may be further adjusted based on the intermediate measurement information.

In some embodiments, when glycated hemoglobin is measured based on ion exchange chromatography, the dosage of the pretreatment test solution entering ionexchange column chromatography may be adjusted based on the intermediate measurement information.

In some other embodiments, when glycated hemoglobin is measured based on affinity chromatography, the dosage of the pretreatment test solution entering the affinity column chromatography may be adjusted based on the intermediate measurement information.

In some alternative or additional embodiments, the pretreatment reagent for preparing the first pretreatment test solution includes a hemolytic agent for lysing erythrocytes in the test blood sample to release hemoglobin, and the intermediate measurement information includes a measurement value related to a hemoglobin concentration of the first pretreatment test solution; or the pretreatment reagent for preparing the first pretreatment test solution includes a diluent for diluting the test blood sample to measure a hematocrit, and the intermediate measurement information includes a measurement value related to the hematocrit of the first pretreatment test solution. The target test item is a glycated hemoglobin test item, and the second test apparatus 130 is further configured to measure glycated hemoglobin in the test blood sample. In this case, the controller 140 is further configured to:
when the measurement value related to the hemoglobin concentration and/or the hematocrit of the first pretreatment test solution are/is greater than a first preset threshold, select a fourth test mode as the test mode to be performed; and
when the measurement value related to the hemoglobin concentration and/or the hematocrit of the first pretreatment test solution are/is less than the first preset threshold, select a fifth test mode as the test mode to be performed, wherein the test sample solution is prepared based on enzymatic assay for glycated hemoglobin under the fourth test mode, and the test sample solution is prepared based on turbidimetric inhibition immunoassay for glycated hemoglobin under the fifth test mode.

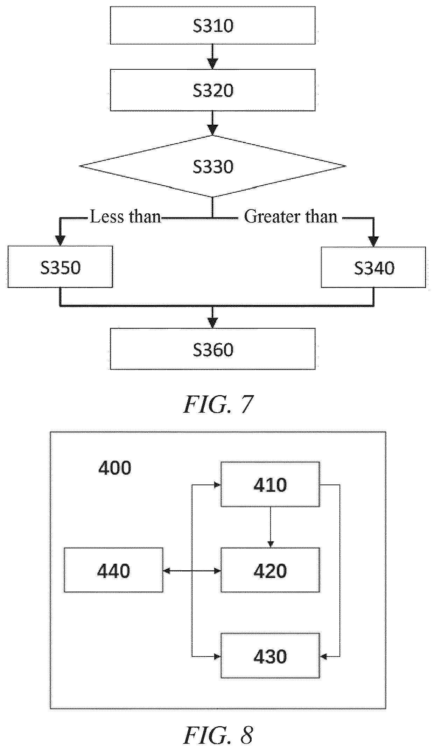

FIG. 7 shows a specific process of a glycated hemoglobin test. In step S310, the sample preparation apparatus 110 mixes a test blood sample with a hemolytic agent to obtain a first pretreatment test solution. In step S320, the first test apparatus 120 detects optical signals in a process of the test blood sample reacting with the hemolytic agent, so as to obtain an estimated hemoglobin concentration of the first pretreatment test solution based on the optical signals. In step S330, the controller 140 determines whether the estimated hemoglobin concentration is greater than the first preset threshold. When the estimated hemoglobin concentration is greater than the first preset threshold, step S340 is performed: the sample preparation apparatus 110 prepares the test sample solution according to the fourth test mode. When the estimated hemoglobin concentration is less than the first preset threshold, step S350 is performed: the sample preparation apparatus 110 prepares the test sample solution according to the fifth test mode. In step S360, the second test apparatus 130 performs a glycated hemoglobin test on the test sample solution.

In a specific example, a hemoglobin measurement range of the enzymatic assay is first determined to be 90 g/L-400 g/L through a plurality of samples with gradient hemoglobin concentrations, wherein an upper limit of measurement is 400 g/L, and a lower limit of measurement is 90 g/L. In addition, likewise, a hemoglobin measurement range of the turbidimetric inhibition immunoassay is determined to be 40 g/L-400 g/L through a plurality of samples with gradient hemoglobin concentrations, wherein an upper limit of measurement is 400 g/L, and a lower limit of measurement is 40 g/L. In a specific testing process, a test whole blood sample is lysed with a hemolytic agent (that is, the pretreatment reagent) according to a certain proportion, so as to obtain a first pretreatment test solution, wherein the hemolytic agent contains a nitrite and other components that can oxidize hemoglobin into methemoglobin. After the hemolytic agent is mixed with the test whole blood sample, the hemoglobin is oxidized into methemoglobin, and an estimated hemoglobin concentration of the first pretreatment test solution may be obtained by measuring absorbance of the first pretreatment test solution at a wavelength of 540 nm or 570 nm. Then, the estimated hemoglobin concentration is compared with upper and lower limits of hemoglobin measurement by the enzymatic assay, and a type of a test reagent involved in the subsequent preparation of a test sample solution is determined. When the estimated hemoglobin concentration is between the upper and lower limits of hemoglobin measurement by the enzymatic assay, a test reagent based on the enzymatic assay is selected to prepare the test sample solution; and when the estimated hemoglobin concentration is less than the lower limit of hemoglobin measurement by the enzymatic assay, a test reagent based on turbidimetric inhibition immunoassay is selected to prepare the test sample solution.

Herein, although the lower limit of glycated hemoglobin measurement by the enzymatic assay is greater than the lower limit of glycated hemoglobin measurement by the turbidimetric inhibition immunoassay, a single test cost of the enzymatic assay for glycated hemoglobin is less than a single test cost of the turbidimetric inhibition immunoassay for glycated hemoglobin. A corresponding test reagent is selected based on a measurement value related to the hemoglobin concentration and/or the hematocrit of the first pretreatment test solution, so that a wider effective measurement range can be provided without increasing costs as much as possible.

In some alternative or additional embodiments, the pretreatment reagent for preparing the first pretreatment test solution includes a hemolytic agent for lysing erythrocytes in the test blood sample to release hemoglobin, and the intermediate measurement information includes a measurement value related to an interferent in the first pretreatment test solution that interferes with the target item test. The target test item is a glycated hemoglobin test item, and the second test apparatus 140 is further configured to measure glycated hemoglobin in the test blood sample. Herein, the controller 140 is further configured to:
when the measurement value related to the interferent in the first pretreatment test solution exceeds a second preset threshold, select a sixth test mode as the test mode to be performed, and control, according to the sixth test mode, the sample preparation apparatus 110 to aspirate a blood sample of the subject again, prepare a new pretreatment test solution based on the blood sample aspirated again and a new pretreatment reagent, and prepare the test sample solution by using the new pretreatment test solution and the test reagent, wherein the new pretreatment reagent contains an anti-interference reagent for eliminating the interferent; and
when the measurement value related to the interferent in the first pretreatment test solution does not exceed the preset threshold, select a seventh test mode as the test mode to be performed, and control, according to the seventh test mode, the sample preparation apparatus 110 to prepare the test sample solution by using the test reagent and the second pretreatment test solution that is from the first pretreatment test solution after being tested by the first test apparatus.

That is, when it is determined, based on the measurement value related to the interferent in the first pretreatment test solution, that there is an interferent, such as lipid interference, that interferes with measurement of glycated hemoglobin in the test blood sample, an anti-interference reagent for eliminating the interferent is added when the test sample solution is subsequently prepared, thereby ensuring that an accurate glycated hemoglobin test result can be obtained.

In this case, the measurement value related to the interferent in the first pretreatment test solution particularly includes a lipemia concentration or a bilirubin concentration.

In some embodiments, the controller 140 is further configured to: when the intermediate measurement information of the first pretreatment test solution meets a preset condition, for example, is less than a lower limit of the effective test range, terminate testing of the current test blood sample, and output an alarm prompt.

FIG. 8 is a schematic block diagram of a sample analyzer 400 according to a second implementation of the disclosure. The sample analyzer 400 includes a sample preparation apparatus 410, a first test apparatus 420, a second test apparatus 430, and a controller 440. The sample preparation apparatus 410 is configured to prepare test sample solutions respectively corresponding to at least two different test modes based on a test blood sample, wherein each test sample solution is prepared from a pretreatment test solution and a test reagent, and the pretreatment test solution is prepared from the test blood sample and a pretreatment reagent. The first test apparatus 420 is configured to test at least one pretreatment test solution, so as to obtain intermediate measurement information of the at least one pretreatment test solution. The second test apparatus 430 is configured to test the test sample solutions respectively corresponding to the test modes, so as to obtain a plurality of test results for a same target test item. The controller 440 is configured to: based on the intermediate measurement information of the at least one pretreatment test solution, select at least one test result from the plurality of test results, and output the at least one test result.

In the second implementation, the plurality of test results for the same test item, such as a glycated hemoglobin test item, are obtained by simultaneously testing the plurality of test sample solutions prepared according to the plurality of test modes, and a test result to be output is selected based on the intermediate measurement information, which can reduce or even avoid retesting.

In some embodiments, the pretreatment reagent for preparing the first pretreatment test solution includes a hemolytic agent for lysing erythrocytes in the test blood sample to release hemoglobin, and the intermediate measurement information includes a measurement value related to a hemoglobin concentration of the pretreatment test solution; or the pretreatment reagent for preparing the first pretreatment test solution includes a diluent for diluting the test blood sample to measure a hematocrit, and the intermediate measurement information includes a measurement value related to a hematocrit of the pretreatment test solution; and the target test item is a glycated hemoglobin test item, and the second test apparatus 140 is further configured to measure glycated hemoglobin in the test blood sample.

In some embodiments, the different test modes include at least two test modes, and effective test ranges of the at least two test modes are at least partially non-overlapping.

Further, the different test modes differ in at least one of the following aspects:
a dosage of at least one of the test blood sample, the pretreatment reagent and the test reagent used to prepare the test sample solution, or in the test sample solution, a proportion of at least one of the test blood sample, the pretreatment reagent and the test reagent used to prepare the test sample solution;
a component, a formula or a dosage of the pretreatment reagent used to prepare the test sample solution;
a type or a dosage of the test reagent used to prepare the test sample solution; and
a methodology for testing the test sample solution for the target test item.

In some embodiments, the sample analyzer 400 may be configured as a biochemical instrument as shown in FIG. 2.

For other features and advantages of the sample analyzer 400 according to the second implementation of the disclosure, reference may be made to the various embodiments and advantages thereof of the above sample analyzer 100 according to the first implementation of the disclosure.

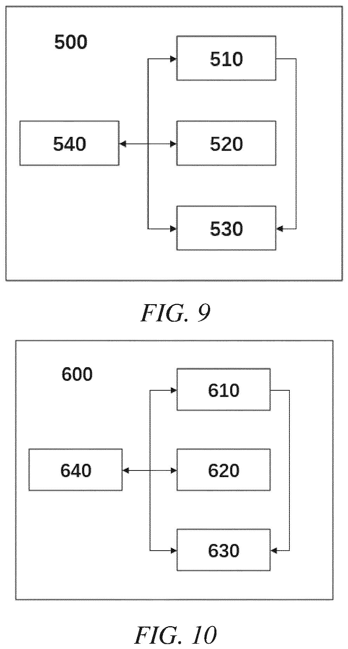

FIG. 9 is a schematic block diagram of a sample analyzer 500 according to a third implementation of the disclosure. The sample analyzer 500 includes a sample preparation apparatus 510, an information obtaining apparatus 520, a second test apparatus 530, and a controller 540. The sample preparation apparatus 510 is configured to prepare, from a test blood sample of a subject, a test sample solution for testing glycated hemoglobin. The information obtaining apparatus 520 is configured to obtain erythrocyte-related information of the subject. The second test apparatus 530 is configured to test the test sample solution, so as to obtain a glycated hemoglobin test result. The controller 540 is configured to:
when an initial test of a glycated hemoglobin item is performed on the test blood sample of the subject, select a test mode to be performed from a plurality of different glycated hemoglobin test modes based on the erythrocyte-related information of the subj ect;
control the sample preparation apparatus 510 to prepare the test sample solution according to the selected test mode to be performed, wherein the test sample solution is prepared based on the test blood sample of the subject, a pretreatment reagent, and a test reagent for testing glycated hemoglobin; and
control the second test apparatus 530 to test the test sample solution.

Therefore, a more accurate glycated hemoglobin test result can be obtained through one test, and retesting of the glycated hemoglobin item can be reduced or even avoided, thereby improving the testing efficiency of the glycated hemoglobin item.

In some embodiments, the erythrocyte-related information of the subject includes hematocrit-related information of the subject. As mentioned above, a glycated hemoglobin concentration of a blood sample has a linear relationship with a hemoglobin concentration of the blood sample, and the hemoglobin concentration of the blood sample also has a linear relationship with a hematocrit (HCT) of the blood sample. Therefore, when the initial test of the glycated hemoglobin item is performed on the test blood sample of the subject, the test mode to be performed may be selected from the plurality of different glycated hemoglobin test modes based on a HCT value of the blood sample of the subject.

In an example, the sample preparation apparatus 510 is configured to perform centrifugation or natural sedimentation on the test blood sample. The information obtaining apparatus 520 is configured as a camera and is configured to image the test blood sample after the centrifugation or natural sedimentation, so as to obtain an image of the test blood sample. The hematocrit of the test blood sample can be obtained based on the image, and then, the test mode to be performed is selected from the plurality of different glycated hemoglobin test modes based on the hematocrit. Preferably, it takes at least half an hour for the natural sedimentation to obtain a better capturing effect. For example, when it is determined, based on the hematocrit of the test blood sample, that the glycated hemoglobin concentration of the test blood sample is relatively low, a test mode in which a sample volume is increased is enabled to increase the glycated hemoglobin concentration in the reaction system, and to reduce the impact of a non-specific background reaction on measurement of the glycated hemoglobin concentration, thereby solving the problem that results are inaccurate during testing of low-concentration glycated hemoglobin, and achieving the effect of obtaining an accurate glycated hemoglobin test result through one test.

In another example, the sample preparation apparatus 510 is configured to aspirate a blood sample from a container 10 containing the test blood sample, such as the whole blood sample, of the subject, and prepare a test sample solution by using the aspirated blood sample, the pretreatment reagent, and the test reagent for testing glycated hemoglobin. Herein, an aspiration pressure of the sample preparation apparatus 510 for aspirating the blood sample is related to a proportion of erythrocytes to whole blood, that is, HCT, and it is known that a concentration of erythrocytes in whole blood (HCT) is positively correlated with a concentration of hemoglobin in whole blood. Therefore, a relationship between aspiration pressure and hemoglobin content can be established. Based on this relationship, a hemoglobin content of the test blood sample may be determined based on the aspiration pressure, so as to guide the subsequent testing of glycated hemoglobin. For example, the sample preparation apparatus 510 includes an aspiration needle for aspirating a blood sample and a sensor for measuring a pressure inside the aspiration needle when aspirating the blood sample, that is, the aspiration pressure. Based on the aspiration pressure measured by the sensor, a dosage of at least one of the test blood sample, the pretreatment reagent and the test reagent used to prepare the test sample solution, or a proportion of at least one of the test blood sample, the pretreatment reagent and the test reagent used to prepare the test sample solution in the test sample solution may be determined, or a component, a formula or a dosage of the pretreatment reagent used to prepare the test sample solution may be determined, or a type or a dosage of the test reagent used to prepare the test sample solution may be determined.

In some embodiments, the controller 540 may be further configured to: when the initial test of the glycated hemoglobin item is performed on the test blood sample of the subject, and before the test mode to be performed is selected, prepare a pretreatment test solution by using the test blood sample and the pretreatment reagent; and control the information obtaining apparatus 520 to test the pretreatment test solution, so as to obtain the intermediate measurement information of the pretreatment test solution as the erythrocyte-related information of the subject. Herein, the information obtaining apparatus 520 is configured, for example, as the above first test apparatus 120.

In some alternative embodiments, the information obtaining apparatus 520 may be further configured to:
receive a user input to obtain the erythrocyte-related information of the subject; or
receive the erythrocyte-related information of the subject from another test instrument communicatively connected to the sample analyzer (for example, receiving a hematocrit from a blood cell analyzer communicatively connected to the sample analyzer); or
obtain the erythrocyte-related information of the subject based on disease information of the subject.

In some embodiments, the controller 540 may be further configured to: select the test mode to be performed from the plurality of different glycated hemoglobin test modes based on the intermediate measurement information of the pretreatment test solution; and control the sample preparation apparatus to prepare, according to the selected test mode to be performed, the test sample solution by using the pretreatment test solution and the test reagent for testing glycated hemoglobin.

For other features and advantages of the sample analyzer 500 according to the third implementation of the disclosure, reference may be made to the various embodiments and advantages thereof of the above sample analyzer 100 and the above sample analyzer 400.

FIG. 10 is a schematic block diagram of a sample analyzer 600 according to a fourth implementation of the disclosure. The sample analyzer 600 includes a sample preparation apparatus 610, an information obtaining apparatus 620, a second test apparatus 630, and a controller 640. The sample preparation apparatus 610 is configured to, under at least two different glycated hemoglobin test modes, prepare test sample solutions corresponding to the respective glycated hemoglobin test modes based on a test blood sample of a subject. The information obtaining apparatus 620 is configured to obtain erythrocyte-related information of the subject. The second test apparatus 630 is configured to separately test the test sample solutions corresponding to the respective glycated hemoglobin test modes, so as to obtain a plurality of glycated hemoglobin test results. The controller 640 is configured to: based on the erythrocyte-related information of the subject, select at least one glycated hemoglobin test result from the plurality of glycated hemoglobin test results, and output the at least one glycated hemoglobin test result.

In some embodiments, the information obtaining apparatus 620 may be further configured to test a pretreatment test solution that is produced in a process of preparing the test sample solutions by the sample preparation apparatus 610, wherein the pretreatment test solution is prepared from the test blood sample and a pretreatment reagent, so as to obtain a measurement value related to a hemoglobin concentration and/or a hematocrit of the pretreatment test solution, as the erythrocyte-related information of the subject.

In some alternative embodiments, the information obtaining apparatus 630 may be further configured to:
receive a user input to obtain the erythrocyte-related information of the subject; or
receive the erythrocyte-related information of the subject from another test instrument communicatively connected to the sample analyzer; or
obtain the erythrocyte-related information of the subject based on disease information of the subject.

For other features and advantages of the sample analyzer 600 according to the fourth implementation of the disclosure, reference may be made to the various embodiments and advantages thereof of the above sample analyzer 100, the above sample analyzer 400, and the above sample analyzer 500.

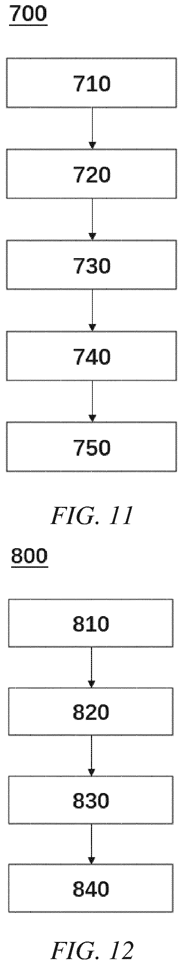

FIG. 11 is a schematic flowchart of a sample testing method 700 according to a fifth implementation of the disclosure. The sample testing method 700 includes the following method steps:
S710: aspirating a blood sample from a container containing a test blood sample of a subject, and performing pretreatment on the aspirated blood sample to obtain a first pretreatment test solution;
S720: testing the first pretreatment test solution to obtain intermediate measurement information of the first pretreatment test solution;
S730: selecting a test mode to be performed from a plurality of different test modes for a same target test item based on the intermediate measurement information of the first pretreatment test solution;
S740: preparing a test sample solution by using a second pretreatment test solution and a test reagent according to the selected test mode to be performed, wherein the second pretreatment test solution is prepared by using the test blood sample of the subject and a pretreatment reagent; and
S750: testing the test sample solution to obtain a test result for the test blood sample for the target test item.

In some embodiments, in step S710, performing pretreatment on the aspirated blood sample to obtain a first pretreatment test solution may include: mixing the aspirated blood sample with a/the pretreatment reagent to obtain the first pretreatment test solution. Preferably, the pretreatment reagent includes a hemolytic agent for lysing erythrocytes in the test blood sample to release hemoglobin, and the intermediate measurement information includes a measurement value related to a hemoglobin concentration of the first pretreatment test solution; or the pretreatment reagent includes a diluent for diluting the test blood sample to measure a hematocrit, and the intermediate measurement information includes a measurement value related to the hematocrit of the first pretreatment test solution.

In some alternative embodiments, in step S710, performing pretreatment on the aspirated blood sample to obtain a first pretreatment test solution may include: performing centrifugation or natural sedimentation on the aspirated blood sample to obtain the first pretreatment test solution. Preferably, the intermediate measurement information includes a measurement value related to a hemoglobin concentration of the first pretreatment test solution or a measurement value related to a hematocrit of the first pretreatment test solution, and the target test item is a glycated hemoglobin test item.

In some embodiments, step S740 of preparing the test sample solution may include: preparing the test sample solution by using the test reagent and the second pretreatment test solution that is from the first pretreatment test solution. That is, the first pretreatment test solution and the second pretreatment test solution are a same pretreatment test solution.

Alternatively, step S740 of preparing the test sample solution may include: aspirating a blood sample of the subject again, preparing the second pretreatment test solution based on the blood sample aspirated again, and preparing the test sample solution based on the second pretreatment test solution prepared and the test reagent.

In some embodiments, the plurality of different test modes may include at least two test modes, and effective test ranges of the at least two test modes are at least partially non-overlapping.

Further, the plurality of different test modes may differ in at least one of the following aspects:
a dosage of at least one of the test blood sample, the pretreatment reagent and the test reagent used to prepare the test sample solution, or in the test sample solution, a proportion of at least one of the test blood sample, the pretreatment reagent and the test reagent used to prepare the test sample solution;
a component, a formula or a dosage of the pretreatment reagent used to prepare the test sample solution;
a type or a dosage of the test reagent used to prepare the test sample solution; and
a methodology for testing the test sample solution for the target test item.

For other features and advantages of the sample testing method 700 according to the fifth implementation of the disclosure, reference may be made to the various embodiments and advantages thereof of the above sample analyzer 100 according to the first implementation of the disclosure.

FIG. 12 is a schematic flowchart of a sample testing method 800 according to a sixth implementation of the disclosure. The sample testing method 800 includes the following method steps:
S810: preparing test sample solutions respectively corresponding to at least two different test modes based on a test blood sample, wherein each test sample solution is prepared from a pretreatment test solution and a test reagent, and the pretreatment test solution is prepared from the test blood sample and a pretreatment reagent;
S820: testing at least one pretreatment test solution to obtain intermediate measurement information of the at least one pretreatment test solution;
S830: testing the test sample solutions respectively corresponding to the test modes, so as to obtain a plurality of test results for a same target test item; and
S840: based on the intermediate measurement information of the at least one pretreatment test solution, selecting at least one test result from the plurality of test results, and outputting the at least one test result.

In some embodiments, the different test modes may include at least two test modes, and effective test ranges of the at least two test modes are at least partially non-overlapping.

Further, the different test modes differ in at least one of the following aspects:
a dosage of at least one of the test blood sample, the pretreatment reagent and the test reagent used to prepare the test sample solution, or in the test sample solution, a proportion of at least one of the test blood sample, the pretreatment reagent and the test reagent used to prepare the test sample solution;
a component, a formula or a dosage of the pretreatment reagent used to prepare the test sample solution;
a type or a dosage of the test reagent used to prepare the test sample solution; and
a methodology for testing the test sample solution for the target test item.

For other features and advantages of the sample testing method 800 according to the sixth implementation of the disclosure, reference may be made to the various embodiments and advantages thereof of the above sample analyzers or the above sample testing methods.

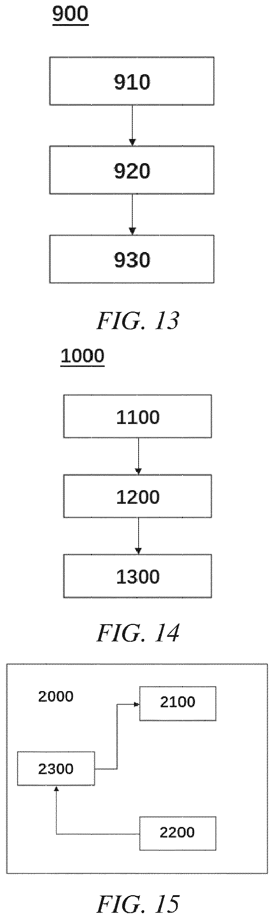

FIG. 13 is a schematic flowchart of a glycated hemoglobin testing method 900 according to a seventh implementation of the disclosure. The glycated hemoglobin testing method 900 includes the following method steps:
S910: when an initial test of a glycated hemoglobin item is performed on a test blood sample of a subject, selecting a test mode to be performed from a plurality of different glycated hemoglobin test modes based on erythrocyte-related information of the subject;
S920: preparing a test sample solution according to the selected test mode to be performed; and
S930: performing a glycated hemoglobin test on the test sample solution.

In some embodiments, the method may further include:
when the initial test of the glycated hemoglobin item is performed on the test blood sample of the subject, and before the test mode to be performed is selected, preparing a pretreatment test solution by using the test blood sample and a pretreatment reagent; and testing the pretreatment test solution to obtain the erythrocyte-related information of the subject.

Alternatively, the method may further include:
receiving a user input to obtain the erythrocyte-related information of the subject; or
obtaining the erythrocyte-related information of the subject based on disease information of the subject.

For other features and advantages of the sample testing method 900 according to the seventh implementation of the disclosure, reference may be made to the various embodiments and advantages thereof of the above sample analyzers or the above sample testing methods.

FIG. 14 is a schematic flowchart of a glycated hemoglobin testing method 1000 according to an eighth implementation of the disclosure. The glycated hemoglobin testing method 1000 includes:
S 1100: under at least two different glycated hemoglobin test modes, preparing test sample solutions corresponding to the respective glycated hemoglobin test modes based on a test blood sample of a subject;
S 1200: separately testing the test sample solutions corresponding to the respective glycated hemoglobin test modes, so as to obtain a plurality of glycated hemoglobin test results; and
S1300: based on erythrocyte-related information of the subject, selecting at least one glycated hemoglobin test result from the plurality of glycated hemoglobin test results, and outputting the at least one glycated hemoglobin test result.

In some embodiments, the method may further include: testing a pretreatment test solution that is produced in a process of preparing the test sample solution, so as to obtain the erythrocyte-related information of the subject.

Alternatively, the method further includes: receiving a user input to obtain the erythrocyte-related information of the subject; or obtaining the erythrocyte-related information of the subject based on disease information of the subject.

For other features and advantages of the sample testing method 1000 according to the eighth implementation of the disclosure, reference may be made to the various embodiments and advantages thereof of the above sample analyzers or the above sample testing methods.

FIG. 15 is a schematic block diagram of a sample analyzer 2000 according to a ninth implementation of the disclosure. The sample analyzer includes a glycated hemoglobin measurement portion 2100, a mode selection portion 2200, and a controller 2300. The glycated hemoglobin measurement portion 2100 is configured to obtain a glycated hemoglobin test result for a blood sample of a subject. The mode selection portion 2200 is configured to select a first glycated hemoglobin test mode or a second glycated hemoglobin test mode. The controller 2300 is configured to:
receive a mode selection of the mode selection portion 2200;
when the mode selection portion 2200 selects the first glycated hemoglobin test mode, control the glycated hemoglobin measurement portion 2100 to execute a first measurement process; and
when the mode selection portion 2200 selects the second glycated hemoglobin test mode, control the glycated hemoglobin measurement portion 2100 to execute a second measurement process, wherein the second measurement process includes: preparing a first pretreatment test solution by using the blood sample of the subject and a pretreatment reagent, testing the first pretreatment test solution to obtain intermediate measurement information, preparing a test sample solution for the second measurement process by using a second pretreatment test solution and a glycated hemoglobin test reagent based on the intermediate measurement information, and testing the test sample solution for the second measurement process to obtain a glycated hemoglobin test result, wherein the second pretreatment solution is prepared from the blood sample of the subject and a/the pretreatment reagent, and the first measurement process and the second measurement process are at least partially different.

Alternatively, the controller 2300 is configured to:
receive a mode selection of the mode selection portion 2200;
when the mode selection portion 2200 selects the first glycated hemoglobin test mode, control the glycated hemoglobin measurement portion 2100 to execute a first measurement process; and
when the mode selection portion 2200 selects the second glycated hemoglobin test mode, control the glycated hemoglobin measurement portion 2100 to execute a second measurement process, wherein an effective test range of one glycated hemoglobin test by the first measurement process is smaller than an effective test range of one glycated hemoglobin test by the second measurement process.

In this embodiment of the disclosure, the sample analyzer may be an analyzer having a single function of glycated hemoglobin test only, or may be an analyzer having multiple functions of glycated hemoglobin test and other test items, such as ISE or other biochemistry test items.

In this embodiment of the disclosure, the effective test range is a variation range of a concentration of an analyte in which test results obtained by a method have precision and accuracy meeting requirements and are linear.

In this embodiment of the disclosure, two types of glycated hemoglobin test modes are provided, so that options of glycated hemoglobin test modes can be increased. In addition, a glycated hemoglobin test mode in which subsequent testing of glycated hemoglobin is guided based on the intermediate measurement information of the pretreatment test solution is provided, so that the accuracy of testing glycated hemoglobin can be improved, retesting can be reduced, and the overall testing efficiency of the sample analyzer can be improved.

In some embodiments, the glycated hemoglobin measurement portion 2100 may include the sample preparation apparatus 110, the first test apparatus 120, and the second test apparatus 130 shown in FIG. 1. The sample preparation apparatus 110 is configured to aspirate a blood sample from the container 10 containing the blood sample of the subject, such as a whole blood sample, and prepare the first pretreatment test solution by using the aspirated blood sample and the pretreatment reagent; and prepare the second pretreatment test solution by using the blood sample of the subject and the pretreatment reagent, and prepare the test sample solution by using the second pretreatment test solution and the glycated hemoglobin test reagent. The first test apparatus 120 is configured to test the first pretreatment test solution prepared by the sample preparation apparatus 110, so as to obtain the intermediate measurement information of the first pretreatment test solution. The second test apparatus 130 is configured to test the test sample solution prepared by the sample preparation apparatus 110, so as to obtain the glycated hemoglobin test result.

In a specific embodiment, the sample analyzer 2000 is configured as a biochemical instrument as shown in FIG. 2. For this, reference may be made to the above description of FIG. 2, and details will not be repeated herein.

Taking turbidimetric inhibition immunoassay and enzymatic assay for measuring glycated hemoglobin as an example, in some embodiments, the controller 2300 may be further configured to: perform the following steps when controlling the glycated hemoglobin measurement portion 2100 to execute the second measurement process:
controlling the sample preparation apparatus 110 to prepare a first test sample solution by using the second pretreatment test solution and a first test reagent for testing glycated hemoglobin, wherein the first test reagent includes an enzyme reagent (such as a proteolytic enzyme, used for acting on glycated hemoglobin in the second pretreatment test solution to produce glycosylated peptides or glycosylated amino acids) or an immunological reagent (such as a glycated hemoglobin-specific antibody) for measuring a hemoglobin content;
controlling the second test apparatus 130 to test the first test sample solution, so as to obtain a hemoglobin content of the blood sample;
controlling the sample preparation apparatus 110 to add a second test reagent for the glycated hemoglobin test item to the first test sample solution to prepare a second test sample solution, wherein the second test reagent includes an enzyme reagent (such as a specific oxidase, used to react with the glycosylated peptide or glycosylated amino acid in the first test sample solution to produce detectable products, such as hydrogen peroxide) or an immunological reagent (such as multi-cluster single-antigens carrying a number of glycated hemoglobin antigenic determinants) for measuring glycated hemoglobin;
controlling the second test apparatus 130 to test the second test sample solution, so as to obtain a glycated hemoglobin content of the blood sample; and
calculating a ratio of the glycated hemoglobin content to the hemoglobin content as a test result for the glycated hemoglobin test item.

In some embodiments, the first test process may include: preparing a test sample solution for the first measurement process by using the blood sample of the subject, the pretreatment reagent, and the glycated hemoglobin test reagent, testing the test sample solution for the first measurement process to obtain a glycated hemoglobin test result, and when the glycated hemoglobin test result is abnormal, preparing a new test sample solution by using the blood sample of the subject, the pretreatment reagent, and the glycated hemoglobin test reagent, and performing a glycated hemoglobin test on the new test sample solution; wherein the test sample solution for the first measurement process and the new test sample solution are prepared differently in terms of dosage or proportion of at least one of the blood sample, the pretreatment reagent and the glycated hemoglobin test reagent; or the test sample solution for the first measurement process and the new test sample solution are prepared differently in terms of component, formula or dosage of the pretreatment reagent; or the test sample solution for the first measurement process and the new test sample solution are prepared differently in terms of type or dosage of the glycated hemoglobin test reagent.

In some embodiments, the mode selection portion 2200 may be further configured to select the first glycated hemoglobin test mode or the second glycated hemoglobin test mode based on erythrocyte-related information of the subject. For example, the mode selection portion may be configured to: receive a user input to obtain the erythrocyte-related information of the subject; or receive the erythrocyte-related information of the subject from another test instrument communicatively connected to the sample analyzer; or obtain the erythrocyte-related information by an information obtaining apparatus, which is provided on the sample analyzer or communicatively connected to the sample analyzer; or obtain the erythrocyte-related information of the subject based on disease information of the subject.

In some embodiments, the mode selection portion 2200 is further configured to select the first glycated hemoglobin test mode or the second glycated hemoglobin test mode based on a user input. For example, the mode selection portion 200 includes a user interface configured to display a control for selecting a glycated hemoglobin test mode and receive an operation on the control, so as to select the first glycated hemoglobin test mode or the second glycated hemoglobin test mode.

In some embodiments, in the second measurement process, preparing a test sample solution for the second measurement process by using a second pretreatment test solution and a glycated hemoglobin test reagent based on the intermediate measurement information may include:
selecting a test mode to be performed from a plurality of different glycated hemoglobin subtest modes based on the intermediate measurement information; and
preparing, according to the test mode to be performed, the test sample solution for the second measurement process by using the second pretreatment test solution and the glycated hemoglobin test reagent.

In a first example, preferably, the first pretreatment test solution and the second pretreatment test solution are a same pretreatment test solution. Therefore, the overall testing efficiency of the sample analyzer can be improved, and in addition, the overall testing cost can be reduced.

Alternatively, in a second example, it may be specified that in the second measurement process, the first pretreatment test solution may not be used to prepare the test sample solution for the second measurement process, but the newly prepared second pretreatment test solution may be used to prepare the test sample solution for the second measurement process.

Alternatively, in a third example, it may be specified that in the second measurement process, the following steps are performed:
under the plurality of different glycated hemoglobin subtest modes, preparing pretreatment test solutions corresponding to the respective glycated hemoglobin subtest modes by using the blood sample of the subject and the pretreatment reagent;
testing at least one pretreatment test solution of the pretreatment test solutions as the first pretreatment test solution, so as to obtain the intermediate measurement information of the first pretreatment test solution;
selecting the test mode to be performed from the plurality of different glycated hemoglobin subtest modes based on the intermediate measurement information; and
according to the test mode to be performed, selecting a pretreatment test solution corresponding to the test mode to be performed from the pretreatment test solutions as the second pretreatment test solution, and preparing the test sample solution for the second measurement process by using the selected pretreatment test solution and the glycated hemoglobin test reagent.

For a specific manner of preparing the test sample solution in the above three examples, reference may be made to the above description of FIG. 2, which will not be repeated herein.

Alternatively, in the second measurement process, preparing a test sample solution for the second measurement process by using a second pretreatment test solution and a glycated hemoglobin test reagent based on the intermediate measurement information includes:
under the plurality of different glycated hemoglobin subtest modes, preparing test sample solutions for the second measurement process corresponding to the respective glycated hemoglobin subtest modes by using the blood sample of the subject, the pretreatment reagent, and the glycated hemoglobin test reagent;
separately testing the test sample solutions for the second measurement process corresponding to the respective glycated hemoglobin subtest modes, so as to obtain a plurality of glycated hemoglobin test results; and
based on the intermediate information, selecting at least one glycated hemoglobin test result from the plurality of glycated hemoglobin test results, and outputting the at least one glycated hemoglobin test result.

In some embodiments, the plurality of different glycated hemoglobin subtest modes may include at least two subtest modes, and effective test ranges of the at least two subtest modes are at least partially non-overlapping. Herein, the intermediate measurement information of the first pretreatment test solution can be used to estimate a test result of the test blood sample for glycated hemoglobin, for example, a hemoglobin concentration or a hematocrit of the first pretreatment test solution can be used to estimate a glycated hemoglobin concentration of the test blood sample, so that a test mode suitable for an effective test range of the test blood sample can be selected based on the intermediate measurement information of the first pretreatment test solution, thereby preventing the test result from exceeding the effective test range, and implementing testing for the glycated hemoglobin through one test.

In some embodiments, the plurality of different glycated hemoglobin subtest modes differ in at least one of the following aspects:
a dosage of at least one of the blood sample, the pretreatment reagent and the glycated hemoglobin test reagent used to prepare the test sample solution for the second measurement process, or in the test sample solution for the second measurement process, a proportion of at least one of the test blood sample, the pretreatment reagent and the glycated hemoglobin test reagent used to prepare the test sample solution for the second measurement process;
a component, a formula or a dosage of the pretreatment reagent used to prepare the test sample solution for the second measurement process; and
a type or a dosage of the glycated hemoglobin test reagent used to prepare the test sample solution for the second measurement process.

Alternatively or additionally, in the second measurement process, it may be further specified that the test sample solution for the second measurement process is tested according to the selected test mode to be performed, so as to obtain a glycated hemoglobin test result for the test blood sample. Herein, the plurality of different glycated hemoglobin subtest modes may differ in at least one of the following aspects:
a wavelength of light used to test glycated hemoglobin in the test sample solution for the second measurement process; and
a methodology used to test glycated hemoglobin in the test sample solution for the second measurement process.

In some embodiments, in the second measurement process, testing the first pretreatment test solution to obtain intermediate measurement information may further include:
irradiating the first pretreatment test solution with a light of at least one wavelength, and obtaining optical signals generated after the first pretreatment test solution is irradiated with the light, so as to obtain the intermediate measurement information based on the optical signals.

For example, as shown in FIG. 3, the first test apparatus 120 may be further configured to irradiate the first pretreatment test solution with a light of at least one wavelength, and obtain optical signals generated after the first pretreatment test solution is irradiated with the light, so as to obtain the intermediate measurement information based on the optical signals.

In some other embodiments, the pretreatment reagent includes a hemolytic agent for lysing erythrocytes in the test blood sample to release hemoglobin. Herein, in the second measurement process, testing the first pretreatment test solution to obtain intermediate measurement information may further include: imaging the first pretreatment test solution to obtain an image of the first pretreatment test solution, and obtaining a measurement value (the intermediate measurement information) related to a hemoglobin concentration of the first pretreatment test solution based on the image.

For example, the first test apparatus 120 is configured as a camera, and the camera is configured to image the first pretreatment test solution to obtain an image of the first pretreatment test solution. The measurement value related to the hemoglobin concentration of the first pretreatment test solution may be obtained based on the image, for example, the hemoglobin concentration of the first pretreatment test solution is estimated based on depth of red in the image.

In still some other embodiments, the pretreatment reagent includes a diluent, and herein, in the second measurement process, testing the first pretreatment test solution to obtain intermediate measurement information may further include: performing centrifugation or natural sedimentation on the first pretreatment test solution, and imaging the first pretreatment test solution after the centrifugation or natural sedimentation, so as to obtain an image of the first pretreatment test solution. The measurement value related to the hemoglobin concentration of the first pretreatment test solution may be obtained based on the image, for example, the glycated hemoglobin concentration of the first pretreatment test solution is estimated based on a proportion of erythrocytes at the bottom to plasma in the upper layer after the centrifugation. In this case, the first test apparatus 120 may be configured as a camera.

In some other alternative embodiments, in the second measurement process, testing the first pretreatment test solution to obtain intermediate measurement information may further include: detecting pulse measurement signals when the first pretreatment test solution passes through a resistance measuring apparatus, so as to obtain the intermediate measurement information based on the pulse measurement signals.

Herein, the first test apparatus 120 is configured, for example, as a resistance measuring apparatus and is used for the first pretreatment test solution to pass through, so as to obtain pulse measurement signals, such that the intermediate measurement information is obtained based on the pulse measurement signals. Herein, preferably, the pretreatment reagent includes a diluent for diluting the test blood sample to measure a hematocrit, and the intermediate measurement information includes a hematocrit of the first pretreatment test solution.

In some embodiments, the pretreatment reagent includes a hemolytic agent for lysing erythrocytes in the blood sample to release hemoglobin, and the intermediate measurement information includes a measurement value related to a hemoglobin concentration of the first pretreatment test solution; or the pretreatment reagent includes a diluent for diluting the blood sample to measure a hematocrit, and the intermediate measurement information includes a measurement value related to a hematocrit of the first pretreatment test solution. Herein, in the second measurement process, the following steps may be performed:
when the measurement value related to the hemoglobin concentration and/or the hematocrit of the first pretreatment test solution are/is within a first preset range, selecting a first subtest mode as the test mode to be performed; and
when the measurement value related to the hemoglobin concentration and/or the hematocrit of the first pretreatment test solution are/is greater than an upper limit of the first preset range, selecting a second subtest mode as the test mode to be performed, wherein in the test sample solution for the second measurement process, a proportion of the blood sample or the second pretreatment test solution used to prepare the test sample solution for the second measurement process under the first subtest mode is greater than a proportion of the blood sample or the second pretreatment test solution used to prepare the test sample solution for the second measurement process under the second subtest mode; and/or
when the measurement value related to the hemoglobin concentration and/or the hematocrit of the first pretreatment test solution are/is less than a lower limit of the first preset range, selecting a third subtest mode as the test mode to be performed, wherein in the test sample solution for the second measurement process, a proportion of the blood sample or the second pretreatment test solution used to prepare the test sample solution for the second measurement process under the first subtest mode is less than a proportion of the blood sample or the second pretreatment test solution used to prepare the test sample solution for the second measurement process under the third subtest mode.

Herein, a dosage of the test blood sample or the second pretreatment test solution used for preparing the test sample solution for the second measurement process may be increased and/or a volume of the test sample solution for the second measurement process may be reduced, so as to increase the proportion of the test blood sample or the second pretreatment test solution used to prepare the test sample solution for the second measurement process in the test sample solution. Likewise, the dosage of the test blood sample or the second pretreatment test solution used for preparing the test sample solution for the second measurement process may be reduced and/or the volume of the test sample solution for the second measurement process may be increased, so as to reduce the proportion of the test blood sample or the second pretreatment test solution used to prepare the test sample solution for the second measurement process in the test sample solution.

In some other examples, a proportion of the dosage of the first pretreatment test solution to the dosage of the test reagent may be further adjusted based on the intermediate measurement information.

In some embodiments, when glycated hemoglobin is measured based on ion exchange chromatography, the dosage of the pretreatment test solution entering ionexchange column chromatography may be adjusted based on the intermediate measurement information.

In some other embodiments, when glycated hemoglobin is measured based on affinity chromatography, the dosage of the pretreatment test solution entering the affinity column chromatography may be adjusted based on the intermediate measurement information.

In some alternative or additional embodiments, in the second measurement process, the following steps may be performed:
when the measurement value related to the hemoglobin concentration and/or the hematocrit of the first pretreatment test solution are/is greater than a first preset threshold, selecting a fourth subtest mode as the test mode to be performed; and
when the measurement value related to the hemoglobin concentration and/or the hematocrit of the first pretreatment test solution are/is less than or equal to the first preset threshold, selecting a fifth subtest mode as the test mode to be performed, wherein the test sample solution for the second measurement process is prepared based on enzymatic assay for glycated hemoglobin under the fourth subtest mode, and the test sample solution for the second measurement process is prepared based on turbidimetric inhibition immunoassay for glycated hemoglobin under the fifth subtest mode.

In some alternative or additional embodiments, in the second measurement process,
the intermediate measurement information includes a measurement value related to an interferent in the first pretreatment test solution that interferes with a glycated hemoglobin test;
when the measurement value related to the interferent in the first pretreatment test solution exceeds a second preset threshold, a sixth subtest mode is selected as the test mode to be performed, and according to the sixth subtest mode, a blood sample of the subject is aspirated again, a new pretreatment test solution is prepared based on the blood sample aspirated again and a new pretreatment reagent, and the test sample solution for the second measurement process is prepared by using the new pretreatment test solution and the glycated hemoglobin test reagent, wherein the new pretreatment reagent contains an anti-interference reagent for eliminating the interferent; and
when the measurement value related to the interferent in the first pretreatment test solution does not exceed the second preset threshold, a seventh subtest mode is selected as the test mode to be performed, and according to the seventh subtest mode, the test sample solution for the second measurement process is prepared by using the glycated hemoglobin test reagent and the second pretreatment test solution that is from the first pretreatment test solution.

That is, when it is determined, based on the measurement value related to the interferent in the first pretreatment test solution, that there is an interferent, such as lipid interference, that interferes with measurement of glycated hemoglobin in the test blood sample, an anti-interference reagent for eliminating the interferent is added when the test sample solution for the second measurement process is subsequently prepared, thereby ensuring that an accurate glycated hemoglobin test result can be obtained.

In this case, the measurement value related to the interferent in the first pretreatment test solution particularly includes a lipemia concentration or a bilirubin concentration.

An example of the disclosure is described by taking measuring glycated hemoglobin based on enzymatic assay as an example. First, a whole blood sample to be tested is lysed with a hemolytic agent (that is, the pretreatment reagent) according to a certain proportion, so as to obtain a first pretreatment test solution, wherein the hemolytic agent contains a nitrite and other components that can oxidize hemoglobin into methemoglobin. After the hemolytic agent is mixed with the whole blood sample to be tested, the hemoglobin is oxidized into methemoglobin, and an estimated lipid concentration of the first pretreatment test solution may be obtained by measuring absorbance of the first pretreatment test solution at a wavelength greater than 600 nm. Then, the estimated lipid concentration is compared with a preset threshold and it is determined whether to add a fat-reducing component when the test sample solution is subsequently prepared. When the lipemia concentration is greater than the set threshold, a whole blood sample to be tested is aspirated again, and the whole blood sample aspirated again is mixed with a hemolytic agent to which a fat-reducing component is added, so as to prepare a second pretreatment test solution. At least part of the second pretreatment test solution is mixed with a first test reagent containing a proteolytic enzyme to obtain a first test sample solution, the proteolytic enzyme acts on glycated hemoglobin in the test solution to produce glycosylated peptide or glycosylated amino acid, absorbance of the first test sample solution at a wavelength of 480 nm is measured at the same time, and a hemoglobin concentration of the test blood sample is obtained based on the absorbance and a calibration curve. Subsequently, the second test reagent containing a specific oxidase is added to the first test sample solution to obtain a second test sample solution, and the specific oxidase reacts with the glycosylated peptide or glycosylated amino acid to produce detectable products (such as the hydrogen peroxide), a chromogenic reaction occurs between the hydrogen peroxide and 10-(carboxymethylaminocarbonyl)-3,7-bis(dimethylamino)phenothiazine sodium (color developing agent), and a glycated hemoglobin concentration is obtained by measuring the absorbance. Finally, a glycated hemoglobin test result is obtained based on the hemoglobin concentration and the glycated hemoglobin concentration.

Alternatively or additionally, in the second measurement process, it may be specified that:
the intermediate measurement information includes a measurement value related to an interferent in the first pretreatment test solution that interferes with a glycated hemoglobin test; and
when the measurement value related to the interferent in the first pretreatment test solution exceeds a third preset threshold, the test sample solution for the second measurement process is prepared based on turbidimetric inhibition immunoassay for glycated hemoglobin.

In a specific example, a whole blood sample to be tested is lysed with a hemolytic agent (that is, the pretreatment reagent) according to a certain proportion, so as to obtain the first pretreatment test solution, wherein the hemolytic agent is used to release hemoglobin by rupturing a erythrocyte membrane. After the hemolytic agent is mixed with the whole blood sample to be tested, the hemoglobin is oxidized into methemoglobin, and absorbance of the first pretreatment test solution at a first wavelength and a second wavelength is measured, the first wavelength is any wavelength within 450 nm-500 nm, and the second wavelength is the absorbance at any wavelength within 500 nm-600 nm, wherein both hemoglobin and bilirubin have characteristic absorption at the first wavelength, while bilirubin has no absorption at the second wavelength, but hemoglobin has characteristic absorption at the second wavelength. Absorbance of bilirubin at the first wavelength can be obtained by subtracting the absorbance of hemoglobin at the first wavelength from the absorbance of the first pretreatment test solution at the first wavelength, and a concentration value of bilirubin in the sample can be obtained by dividing the absorbance of bilirubin at the first wavelength by an extinction coefficient of bilirubin at the wavelength. When the bilirubin concentration is greater than a preset threshold, a test reagent based on turbidimetric inhibition immunoassay is selected to prepare the test sample solution.

In some embodiments, the second measurement process may further include: when the intermediate measurement information exceeds a second preset range, terminating the second measurement process and outputting an alarm prompt. Herein, if the intermediate measurement information exceeds the second preset range, it indicates that a glycated hemoglobin concentration of the current test blood sample is beyond an effective test range of the glycated hemoglobin analyzer. Therefore, even if the test is continued, an accurate glycated hemoglobin concentration cannot be obtained. Terminating the measurement process in advance can further avoid waste of reagents and improve testing efficiency.

For other features and advantages of the sample analyzer 2000 according to the ninth implementation of the disclosure, reference may be made to the various embodiments and advantages thereof of the above sample analyzer 100.

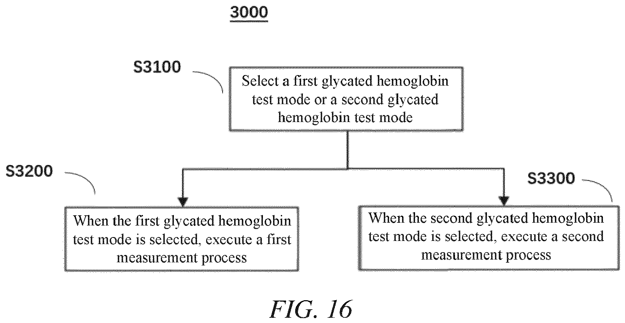

FIG. 16 is a schematic flowchart of a sample testing method according to a tenth implementation of the disclosure. As shown in FIG. 16, the sample testing method 3000 includes the following method steps:
S3100: selecting a first glycated hemoglobin test mode or a second glycated hemoglobin test mode;
S3200: when the first glycated hemoglobin test mode is selected, executing a first measurement process; and
S3300: when the second glycated hemoglobin test mode is selected, executing a second measurement process, wherein the second measurement process includes: preparing a first pretreatment test solution by using a blood sample of a subject and a pretreatment reagent, testing the first pretreatment test solution to obtain intermediate measurement information, preparing a test sample solution for the second measurement process by using a second pretreatment test solution and a glycated hemoglobin test reagent based on the intermediate measurement information, and testing the test sample solution for the second measurement process to obtain a glycated hemoglobin test result, wherein the second pretreatment solution is prepared from the test blood sample of the subject and a/the pretreatment reagent, and the first measurement process and the second measurement process are at least partially different.

In some embodiments, the first test process may include:
preparing a test sample solution for the first measurement process by using the blood sample of the subject, the pretreatment reagent, and the glycated hemoglobin test reagent, testing the test sample solution for the first measurement process to obtain a glycated hemoglobin test result, and when the glycated hemoglobin test result is abnormal, preparing a new test sample solution by using the blood sample of the subject, the pretreatment reagent, and the glycated hemoglobin test reagent, and performing a glycated hemoglobin test on the new test sample solution; wherein the test sample solution for the first measurement process and the new test sample solution are prepared differently in terms of dosage or proportion of at least one of the blood sample, the pretreatment reagent and the glycated hemoglobin test reagent; or the test sample solution for the first measurement process and the new test sample solution are prepared differently in terms of component, formula or dosage of the pretreatment reagent; or the test sample solution for the first measurement process and the new test sample solution are prepared differently in terms of type or dosage of the glycated hemoglobin test reagent.

In some embodiments, in step S3100, selecting a first glycated hemoglobin test mode or a second glycated hemoglobin test mode includes:
selecting the first glycated hemoglobin test mode or the second glycated hemoglobin test mode based on erythrocyte-related information of the subject.

In some embodiments, in the second measurement process, preparing a test sample solution for the second measurement process by using a second pretreatment test solution and a glycated hemoglobin test reagent based on the intermediate measurement information includes:
selecting a test mode to be performed from a plurality of different glycated hemoglobin subtest modes based on the intermediate measurement information; and
preparing, according to the test mode to be performed, the test sample solution for the second measurement process by using the second pretreatment test solution and the glycated hemoglobin test reagent.

In some alternative embodiments, in the second measurement process, the following steps may be performed:
under the plurality of different glycated hemoglobin subtest modes, preparing pretreatment test solutions corresponding to the respective glycated hemoglobin subtest modes by using the blood sample of the subject and the pretreatment reagent;
testing at least one pretreatment test solution of the pretreatment test solutions as the first pretreatment test solution, so as to obtain the intermediate measurement information of the first pretreatment test solution;
selecting the test mode to be performed from the plurality of different glycated hemoglobin subtest modes based on the intermediate measurement information; and
according to the test mode to be performed, selecting a pretreatment test solution corresponding to the test mode to be performed from the pretreatment test solutions as the second pretreatment test solution, and preparing the test sample solution for the second measurement process by using the selected pretreatment test solution and the glycated hemoglobin test reagent.

In some other alternative embodiments, in the second measurement process, preparing a test sample solution for the second measurement process by using a second pretreatment test solution and a glycated hemoglobin test reagent based on the intermediate measurement information may include:
under the plurality of different glycated hemoglobin subtest modes, preparing test sample solutions for the second measurement process corresponding to the respective glycated hemoglobin subtest modes by using the blood sample of the subject, the pretreatment reagent, and the glycated hemoglobin test reagent;
separately testing the test sample solutions for the second measurement process corresponding to the respective glycated hemoglobin subtest modes, so as to obtain a plurality of glycated hemoglobin test results; and
based on the intermediate information, selecting at least one glycated hemoglobin test result from the plurality of glycated hemoglobin test results, and outputting the at least one glycated hemoglobin test result.

In some embodiments, the plurality of different glycated hemoglobin subtest modes may differ in at least one of the following aspects:
a dosage of at least one of the blood sample, the pretreatment reagent and the glycated hemoglobin test reagent used to prepare the test sample solution for the second measurement process, or in the test sample solution for the second measurement process, a proportion of at least one of the test blood sample, the pretreatment reagent and the glycated hemoglobin test reagent used to prepare the test sample solution for the second measurement process;
a component, a formula or a dosage of the pretreatment reagent used to prepare the test sample solution for the second measurement process; and
a type or a dosage of the glycated hemoglobin test reagent used to prepare the test sample solution for the second measurement process.

In some embodiments, the pretreatment reagent may include a hemolytic agent for lysing erythrocytes in the blood sample to release hemoglobin, and the intermediate measurement information includes a measurement value related to a hemoglobin concentration of the first pretreatment test solution.

Alternatively, the pretreatment reagent may include a diluent for diluting the blood sample to measure a hematocrit, and the intermediate measurement information includes a measurement value related to a hematocrit of the first pretreatment test solution.

In some embodiments, in the second measurement process, the following steps may be performed:
when the measurement value related to the hemoglobin concentration and/or the hematocrit of the first pretreatment test solution are/is within a first preset range, selecting a first subtest mode as the test mode to be performed; and
when the measurement value related to the hemoglobin concentration and/or the hematocrit of the first pretreatment test solution are/is greater than an upper limit of the first preset range, selecting a second subtest mode as the test mode to be performed, wherein in the test sample solution for the second measurement process, a proportion of the blood sample or the second pretreatment test solution used to prepare the test sample solution for the second measurement process under the first subtest mode is greater than a proportion of the blood sample or the second pretreatment test solution used to prepare the test sample solution for the second measurement process under the second subtest mode; and/or
when the measurement value related to the hemoglobin concentration and/or the hematocrit of the first pretreatment test solution are/is less than a lower limit of the first preset range, selecting a third subtest mode as the test mode to be performed, wherein in the test sample solution, a proportion of the blood sample or the second pretreatment test solution used to prepare the test sample solution for the second measurement process under the first subtest mode is less than a proportion of the blood sample or the second pretreatment test solution used to prepare the test sample solution for the second measurement process under the third subtest mode.

Alternatively or additionally, in the second measurement process, the following steps may be performed:
when the measurement value related to the hemoglobin concentration and/or the hematocrit of the first pretreatment test solution are/is greater than a first preset threshold, selecting a fourth subtest mode as the test mode to be performed; and
when the measurement value related to the hemoglobin concentration and/or the hematocrit of the first pretreatment test solution are/is less than or equal to the first preset threshold, selecting a fifth subtest mode as the test mode to be performed, wherein the test sample solution for the second measurement process is prepared based on enzymatic assay for glycated hemoglobin under the fourth subtest mode, and the test sample solution for the second measurement process is prepared based on turbidimetric inhibition immunoassay for glycated hemoglobin under the fifth subtest mode.

Alternatively or additionally, the intermediate measurement information includes a measurement value related to an interferent in the first pretreatment test solution that interferes with a glycated hemoglobin test, and the following steps may be performed in the second measurement process:
when the measurement value related to the interferent in the first pretreatment test solution exceeds a second preset threshold, selecting a sixth subtest mode as the test mode to be performed, and according to the sixth subtest mode, aspirating a blood sample of the subject again, preparing a new pretreatment test solution based on the blood sample aspirated again and a new pretreatment reagent, and preparing the test sample solution for the second measurement process by using the new pretreatment test solution and the glycated hemoglobin test reagent, wherein the new pretreatment reagent contains an anti-interference reagent for eliminating the interferent; and
when the measurement value related to the interferent in the first pretreatment test solution does not exceed the second preset threshold, selecting a seventh subtest mode as the test mode to be performed, and according to the seventh subtest mode, preparing the test sample solution for the second measurement process by using the glycated hemoglobin test reagent and the second pretreatment test solution that is from the first pretreatment test solution.

In some embodiments, the second measurement process may further include: when the intermediate measurement information exceeds a third preset range, terminating the second measurement process and outputting an alarm prompt.

For other features and advantages of the sample testing method 3000 provided in this embodiment of the disclosure, reference may be made to the various embodiments and advantages thereof of the above sample analyzer 2000, which will not be repeated herein.

The features or combinations thereof mentioned above in the description, accompanying drawings, and claims may be combined with each other arbitrarily or used separately as long as they are meaningful within the scope of the disclosure and do not contradict each other. The advantages and features described for the sample analyzers provided in the embodiments of the disclosure are applicable in a corresponding manner to the testing methods provided in the embodiments of the disclosure, and vice versa.

The foregoing description merely relates to the preferred embodiments of the disclosure, and is not intended to limit the scope of patent of the disclosure. All equivalent variations made by using the content of the specification and the accompanying drawings of the disclosure from the concept of the disclosure, or the direct/indirect applications of the contents in other related technical fields all fall within the scope of patent protection of the disclosure.

### EXAMPLES

1. A sample analyzer, characterized in that the sample analyzer comprises:
   a sample preparation apparatus configured to aspirate a blood sample from a container containing a test blood sample of a subject, and prepare a first pretreatment test solution by using the aspirated blood sample and a pretreatment reagent;
   a first test apparatus configured to test the first pretreatment test solution, so as to obtain intermediate measurement information of the first pretreatment test solution;
   a controller configured to select a test mode to be performed from a plurality of different test modes based on the intermediate measurement information, and control the sample preparation apparatus to prepare a test sample solution by using a second pretreatment test solution and a test reagent according to the selected test mode to be performed, wherein the second pretreatment test solution is prepared by the sample preparation apparatus using the test blood sample of the subject and a/the pretreatment reagent; and
   a second test apparatus configured to test the test sample solution, so as to obtain a test result of the test blood sample for a target test item.
2. The sample analyzer of example 1, characterized in that the pretreatment reagent for preparing the first pretreatment test solution comprises a hemolytic agent for lysing erythrocytes in the test blood sample to release hemoglobin, and the intermediate measurement information comprises a measurement value related to a hemoglobin concentration of the first pretreatment test solution.
3. The sample analyzer of example 1, characterized in that the pretreatment reagent for preparing the first pretreatment test solution comprises a diluent for diluting the test blood sample to measure a hematocrit, and the intermediate measurement information comprises a measurement value related to a hematocrit of the first pretreatment test solution.
4. The sample analyzer of example 2 or 3, characterized in that the target test item is a glycated hemoglobin test item, and the second test apparatus is further configured to measure glycated hemoglobin in the test blood sample.
5. The sample analyzer of example 4, characterized in that the controller is further configured to:
   control the sample preparation apparatus to prepare a first test sample solution by using the second pretreatment test solution and a first test reagent for the glycated hemoglobin test item according to the selected test mode to be performed, wherein the first test reagent comprises an enzyme reagent or an immunological reagent for measuring a hemoglobin content;
   control the second test apparatus to test the first test sample solution, so as to obtain a hemoglobin content of the test blood sample;
   control the sample preparation apparatus to add a second test reagent for the glycated hemoglobin test item to the first test sample solution, so as to prepare a second test sample solution, wherein the second test reagent comprises an enzyme reagent or an immunological reagent for measuring glycated hemoglobin;
   control the second test apparatus to test the second test sample solution, so as to obtain a glycated hemoglobin content of the test blood sample; and
   calculate a ratio of the glycated hemoglobin content to the hemoglobin content as a test result for the glycated hemoglobin test item.
6. The sample analyzer of any one of examples 1 to 5, characterized in that the first test apparatus is further configured to irradiate the first pretreatment test solution with a light of at least one wavelength, and obtain optical signals generated after the first pretreatment test solution is irradiated with the light, so as to obtain the intermediate measurement information based on the optical signals; or
   the first test apparatus is further configured to allow the first pretreatment test solution to pass through for obtaining pulse measurement signals, so as to obtain the intermediate measurement information based on the pulse measurement signals.
7. The sample analyzer of any one of examples 1 to 6, characterized in that the first test apparatus and the second test apparatus are a same test apparatus.
8. The sample analyzer of any one of examples 1 to 7, characterized in that the first pretreatment test solution and the second pretreatment test solution are a same pretreatment test solution.
9. The sample analyzer of any one of examples 1 to 7, characterized in that the controller is further configured to: when the sample preparation apparatus is controlled to prepare the test sample solution by using the second pretreatment test solution and the test reagent according to the selected test mode to be performed, control the sample preparation apparatus to aspirate a blood sample of the subject again, prepare the second pretreatment test solution based on the blood sample aspirated again, and prepare the test sample solution based on the second pretreatment test solution prepared and the test reagent.
10. The sample analyzer of any one of examples 1 to 7, characterized in that the sample preparation apparatus is further configured to prepare a plurality of pretreatment test solutions by using the test blood sample and the pretreatment reagent, wherein the plurality of pretreatment test solutions are prepared differently from each other in terms of at least one of component, formula and dosage of the pretreatment reagent, and/or the plurality of pretreatment test solutions are prepared differently from each other in terms of dosage of the test blood sample;
   the first test apparatus is further configured to test at least one first pretreatment test solution of the plurality of pretreatment test solutions, so as to obtain the intermediate measurement information of the first pretreatment test solution; and
   the controller is further configured to: when the sample preparation apparatus is controlled to prepare the test sample solution by using the second pretreatment test solution and the test reagent according to the selected test mode to be performed, control the sample preparation apparatus to select one pretreatment test solution from the plurality of pretreatment test solutions as the second pretreatment test solution according to the selected test mode to be performed, and prepare the test sample solution by using the selected pretreatment test solution and the test reagent.
11. The sample analyzer of any one of examples 1 to 10, characterized in that the plurality of different test modes comprise at least two test modes, and effective test ranges of the at least two test modes are at least partially non-overlapping.
12. The sample analyzer of any one of examples 1 to 11, characterized in that the plurality of different test modes differ in at least one of the following aspects:
   a dosage of at least one of the test blood sample, the pretreatment reagent and the test reagent used to prepare the test sample solution, or in the test sample solution, a proportion of at least one of the test blood sample, the pretreatment reagent and the test reagent used to prepare the test sample solution;
   a component, a formula or a dosage of the pretreatment reagent used to prepare the test sample solution;
   a type or a dosage of the test reagent used to prepare the test sample solution; and
   a methodology for testing the test sample solution for the target test item.
13. The sample analyzer of any one of examples 1 to 12, characterized in that the pretreatment reagent for preparing the first pretreatment test solution comprises a hemolytic agent for lysing erythrocytes in the test blood sample to release hemoglobin, and the intermediate measurement information comprises a measurement value related to a hemoglobin concentration of the first pretreatment test solution; or the pretreatment reagent for preparing the first pretreatment test solution comprises a diluent for diluting the test blood sample to measure a hematocrit, and the intermediate measurement information comprises a measurement value related to a hematocrit of the first pretreatment test solution;
   the target test item is a glycated hemoglobin test item, and the second test apparatus is further configured to measure glycated hemoglobin in the test blood sample; and
   the controller is further configured to:
      when the measurement value related to the hemoglobin concentration and/or the hematocrit of the first pretreatment test solution are/is within a preset range, select a first test mode as the test mode to be performed; and
      when the measurement value related to the hemoglobin concentration and/or the hematocrit of the first pretreatment test solution are/is greater than an upper limit of the preset range, select a second test mode as the test mode to be performed, wherein, in the test sample solution, a proportion of the test blood sample or the second pretreatment test solution used to prepare the test sample solution under the first test mode is greater than a proportion of the test blood sample or the second pretreatment test solution used to prepare the test sample solution under the second test mode; and/or
      when the measurement value related to the hemoglobin concentration and/or the hematocrit of the first pretreatment test solution are/is less than a lower limit of the preset range, select a third test mode as the test mode to be performed, wherein, in the test sample solution, the proportion of the test blood sample or the second pretreatment test solution used to prepare the test sample solution under the first test mode is less than a proportion of the test blood sample or the second pretreatment test solution used to prepare the test sample solution under the third test mode.
14. The sample analyzer of any one of examples 1 to 13, characterized in that the pretreatment reagent for preparing the first pretreatment test solution comprises a hemolytic agent for lysing erythrocytes in the test blood sample to release hemoglobin, and the intermediate measurement information comprises a measurement value related to a hemoglobin concentration of the first pretreatment test solution; or the pretreatment reagent for preparing the first pretreatment test solution comprises a diluent for diluting the test blood sample to measure a hematocrit, and the intermediate measurement information comprises a measurement value related to a hematocrit of the first pretreatment test solution;
   the target test item is a glycated hemoglobin test item, and the second test apparatus is further configured to measure glycated hemoglobin in the test blood sample; and
   the controller is further configured to:
      when the measurement value related to the hemoglobin concentration and/or the hematocrit of the first pretreatment test solution are/is greater than a first preset threshold, select a fourth test mode as the test mode to be performed; and
      when the measurement value related to the hemoglobin concentration and/or the hematocrit of the pretreatment test solution are/is less than the first preset threshold, select a fifth test mode as the test mode to be performed, wherein the test sample solution is prepared based on enzymatic assay for glycated hemoglobin under the fourth test mode, and the test sample solution is prepared based on turbidimetric inhibition immunoassay for glycated hemoglobin under the fifth test mode.
15. The sample analyzer of any one of examples 1 to 14, characterized in that the pretreatment reagent for preparing the first pretreatment test solution comprises a hemolytic agent for lysing erythrocytes in the test blood sample to release hemoglobin, and the intermediate measurement information comprises a measurement value related to an interferent in the first pretreatment test solution that interferes with the target test item;
   the target test item is a glycated hemoglobin test item, and the second test apparatus is further configured to measure glycated hemoglobin in the test blood sample; and
   the controller is further configured to:
      when the measurement value related to the interferent in the first pretreatment test solution exceeds a second preset threshold, select a sixth test mode as the test mode to be performed, and control, according to the sixth test mode, the sample preparation apparatus to aspirate a blood sample of the subject again , prepare a new pretreatment test solution based on the blood sample aspirated again and a new pretreatment reagent, and prepare the test sample solution by using the new pretreatment test solution and the test reagent, wherein the new pretreatment reagent contains an anti-interference reagent for eliminating the interferent; and
      when the measurement value related to the interferent in the first pretreatment test solution does not exceed the second preset threshold, select a seventh test mode as the test mode to be performed, and control, according to the seventh test mode, the sample preparation apparatus to prepare the test sample solution by using the test reagent and the second pretreatment test solution that is from the first pretreatment test solution tested by the first test apparatus.
16. A sample analyzer, comprising:
   a sample preparation apparatus configured to prepare test sample solutions respectively corresponding to at least two different test modes based on a test blood sample, wherein each test sample solution is prepared from a pretreatment test solution and a test reagent, and the pretreatment test solution is prepared from the test blood sample and a pretreatment reagent;
   a first test apparatus configured to test at least one pretreatment test solution, so as to obtain intermediate measurement information of the at least one pretreatment test solution;
   a second test apparatus configured to test the test sample solutions respectively corresponding to the test modes, so as to obtain a plurality of test results for a same target test item; and
   a controller configured to: based on the intermediate measurement information of the at least one pretreatment test solution, select at least one test result from the plurality of test results, and output the at least one test result.
17. The sample analyzer of example 16, characterized in that the pretreatment reagent comprises a hemolytic agent for lysing erythrocytes in the test blood sample to release hemoglobin, and the intermediate measurement information comprises a measurement value related to a hemoglobin concentration of the pretreatment test solution; or the pretreatment reagent comprises a diluent for diluting the test blood sample to measure a hematocrit, and the intermediate measurement information comprises a measurement value related to a hematocrit of the pretreatment test solution; and
   the target test item is a glycated hemoglobin test item, and the second test apparatus is further configured to measure glycated hemoglobin in the test blood sample.
18. The sample analyzer of example 16 or 17, characterized in that the different test modes comprise at least two test modes, and effective test ranges of the at least two test modes are at least partially non-overlapping.
19. The sample analyzer of any one of examples 16 to 18, characterized in that the at least two different test modes differ in at least one of the following aspects:
   a dosage of at least one of the test blood sample, the pretreatment reagent and the test reagent used to prepare the test sample solution, or in the test sample solution, a proportion of at least one of the test blood sample, the pretreatment reagent and the test reagent used to prepare the test sample solution;
   a component, a formula or a dosage of the pretreatment reagent used to prepare the test sample solution;
   a type or a dosage of the test reagent used to prepare the test sample solution; and
   a methodology for testing the test sample solution for the target test item.
20. A sample analyzer, comprising:
   a sample preparation apparatus configured to prepare, from a test blood sample of a subject, a test sample solution for testing glycated hemoglobin;
   an information obtaining apparatus configured to obtain erythrocyte-related information of the subject;
   a second test apparatus configured to test the test sample solution, so as to obtain a glycated hemoglobin test result; and
   a controller configured to:
      when an initial test of a glycated hemoglobin item is performed on the test blood sample of the subject, select a test mode to be performed from a plurality of different glycated hemoglobin test modes based on the erythrocyte-related information of the subj ect;
      control the sample preparation apparatus to prepare the test sample solution according to the selected test mode to be performed, wherein the test sample solution is prepared based on the test blood sample of the subject, a pretreatment reagent, and a test reagent for testing glycated hemoglobin; and
      control the second test apparatus to test the test sample solution.
21. The sample analyzer of example 20, characterized in that the controller is further configured to:
   when the initial test of the glycated hemoglobin item is performed on the test blood sample of the subject, and before the test mode to be performed is selected, prepare a pretreatment test solution by using the test blood sample and the pretreatment reagent; and
   control the information obtaining apparatus to test the pretreatment test solution, to obtain the erythrocyte-related information of the subject.
22. The sample analyzer of example 20, characterized in that the information obtaining apparatus is further configured to:
   receive a user input to obtain the erythrocyte-related information of the subject; or
   receive the erythrocyte-related information of the subject from another test instrument communicatively connected to the sample analyzer; or
   obtain the erythrocyte-related information of the subject based on disease information of the subject.
23. The sample analyzer of example 21, characterized in that the controller is further configured to:
   select the test mode to be performed from the plurality of different glycated hemoglobin test modes based on the erythrocyte-related information of the subject; and
   control the sample preparation apparatus to prepare, according to the selected test mode to be performed, the test sample solution by using the pretreatment test solution and the test reagent for testing glycated hemoglobin.
24. A sample analyzer, comprising:
   a sample preparation apparatus configured to: under at least two different glycated hemoglobin test modes, prepare test sample solutions corresponding to the respective glycated hemoglobin test modes based on a test blood sample of a subject;
   an information obtaining apparatus configured to obtain erythrocyte-related information of the subject;
   a second test apparatus configured to separately test the test sample solutions corresponding to the respective glycated hemoglobin test modes, so as to obtain a plurality of glycated hemoglobin test results; and
   a controller configured to: based on the erythrocyte-related information of the subject, select at least one glycated hemoglobin test result from the plurality of glycated hemoglobin test results, and output the at least one glycated hemoglobin test result.
25. The sample analyzer of example 24, characterized in that the information obtaining apparatus is further configured to test a pretreatment test solution that is produced in a process of preparing the test sample solutions by the sample preparation apparatus, wherein the pretreatment test solution is prepared from the test blood sample and a pretreatment reagent, so as to obtain a measurement value related to a hemoglobin concentration and/or a hematocrit of the pretreatment test solution, as the erythrocyte-related information of the subject; or
   the information obtaining apparatus is further configured to:
   receive a user input to obtain the erythrocyte-related information of the subject; or
   receive the erythrocyte-related information of the subject from another test instrument communicatively connected to the sample analyzer; or
   obtain the erythrocyte-related information of the subject based on disease information of the subject.
26. A sample testing method, comprising:
   aspirating a blood sample from a container containing a test blood sample of a subject, and performing pretreatment on the aspirated blood sample to obtain a first pretreatment test solution;
   testing the first pretreatment test solution to obtain intermediate measurement information of the first pretreatment test solution;
   selecting a test mode to be performed from a plurality of different test modes for a same target test item based on the intermediate measurement information of the first pretreatment test solution;
   preparing a test sample solution by using a second pretreatment test solution and a test reagent according to the selected test mode to be performed, wherein the second pretreatment test solution is prepared by using the test blood sample of the subject and a pretreatment reagent; and
   testing the test sample solution to obtain a test result for the test blood sample for the target test item.
27. The sample testing method of example 26, characterized in that performing pretreatment on the aspirated blood sample to obtain a first pretreatment test solution comprises: mixing the aspirated blood sample with a/the pretreatment reagent to obtain the first pretreatment test solution,
   wherein the pretreatment reagent for preparing the first pretreatment test solution comprises a hemolytic agent for lysing erythrocytes in the test blood sample to release hemoglobin, and the intermediate measurement information comprises a measurement value related to a hemoglobin concentration of the first pretreatment test solution; or the pretreatment reagent for preparing the first pretreatment test solution comprises a diluent for diluting the test blood sample to measure a hematocrit, and the intermediate measurement information comprises a measurement value related to a hematocrit of the first pretreatment test solution.
28. The sample testing method of example 26, characterized in that performing pretreatment on the aspirated blood sample to obtain a first pretreatment test solution comprises: centrifuging the aspirated blood sample to obtain the first pretreatment test solution,
   wherein the intermediate measurement information comprises a measurement value related to a hematocrit of the first pretreatment test solution, and the target test item is a glycated hemoglobin test item.
29. The sample testing method of example 27, characterized in that the first pretreatment test solution and the second pretreatment test solution are a same pretreatment test solution; or
   preparing the test sample solution comprises: aspirating a blood sample of the subj ect again, preparing the second pretreatment test solution based on the blood sample aspirated again, and preparing the test sample solution based on the second pretreatment test solution prepared and the test reagent.
30. The sample testing method of any one of examples 26 to 29, characterized in that the plurality of different test modes comprise at least two test modes, and effective test ranges of the at least two test modes are at least partially non-overlapping;
   and preferably, the plurality of different test modes differ in at least one of the following aspects:
   a dosage of at least one of the test blood sample, the pretreatment reagent and the test reagent used to prepare the test sample solution, or in the test sample solution, a proportion of at least one of the test blood sample, the pretreatment reagent and the test reagent used to prepare the test sample solution;
   a component, a formula or a dosage of the pretreatment reagent used to prepare the test sample solution;
   a type or a dosage of the test reagent used to prepare the test sample solution;
      and
   a methodology for testing the test sample solution for the target test item.
31. A sample testing method, comprising:
   preparing test sample solutions respectively corresponding to at least two different test modes based on a test blood sample, wherein each test sample solution is prepared from a pretreatment test solution and a test reagent, and the pretreatment test solution is prepared from the test blood sample and a pretreatment reagent;
   testing at least one pretreatment test solution to obtain intermediate measurement information of the at least one pretreatment test solution;
   testing the test sample solutions respectively corresponding to the test modes, so as to obtain a plurality of test results for a same target test item; and
   based on the intermediate measurement information of the at least one pretreatment test solution, selecting at least one test result from the plurality of test results, and outputting the at least one test result.
32. The sample testing method of example 31, characterized in that the different test modes comprise at least two test modes, and effective test ranges of the at least two test modes are at least partially non-overlapping;
   and preferably, the different test modes differ in at least one of the following aspects:
   a dosage of at least one of the test blood sample, the pretreatment reagent and the test reagent used to prepare the test sample solution, or in the test sample solution, a proportion of at least one of the test blood sample, the pretreatment reagent and the test reagent used to prepare the test sample solution;
   a component, a formula or a dosage of the pretreatment reagent used to prepare the test sample solution;
   a type or a dosage of the test reagent used to prepare the test sample solution; and
   a methodology for testing the test sample solution for the target test item.
33. A glycated hemoglobin testing method, comprising:
   when an initial test of a glycated hemoglobin item is performed on a test blood sample of a subject, selecting a test mode to be performed from a plurality of different glycated hemoglobin test modes based on erythrocyte-related information of the subject;
   preparing a test sample solution according to the selected test mode to be performed; and
   performing a glycated hemoglobin test on the test sample solution.
34. The glycated hemoglobin testing method of example 33, characterized in that the method further comprises:
   when the initial test of the glycated hemoglobin item is performed on the test blood sample of the subject, and before the test mode to be performed is selected, preparing a pretreatment test solution by using the test blood sample and a pretreatment reagent; and testing the pretreatment test solution to obtain the erythrocyte-related information of the subject; or
   the method further comprises:
      receiving a user input to obtain the erythrocyte-related information of the subject; or
      obtaining the erythrocyte-related information of the subject based on disease information of the subject.
35. A glycated hemoglobin testing method, comprising:
   under at least two different glycated hemoglobin test modes, preparing test sample solutions corresponding to the respective glycated hemoglobin test modes based on a test blood sample of a subject;
   separately testing the test sample solutions corresponding to the respective glycated hemoglobin test modes, so as to obtain a plurality of glycated hemoglobin test results; and
   based on erythrocyte-related information of the subject, selecting at least one glycated hemoglobin test result from the plurality of glycated hemoglobin test results, and outputting the at least one glycated hemoglobin test result.
36. The glycated hemoglobin testing method of example 35, characterized in that the method further comprises:
   testing a pretreatment test solution that is produced in a process of preparing the test sample solution, so as to obtain the erythrocyte-related information of the subject; or
   the method further comprises:
      receiving a user input to obtain the erythrocyte-related information of the subject; or
      obtaining the erythrocyte-related information of the subject based on disease information of the subject.

## Claims

1. A sample analyzer, **characterized in that** the sample analyzer comprises:
a sample preparation apparatus configured to aspirate a blood sample from a container containing a test blood sample of a subject, and prepare a first pretreatment test solution by using the aspirated blood sample and a pretreatment reagent;
a first test apparatus configured to test the first pretreatment test solution, so as to obtain intermediate measurement information of the first pretreatment test solution;
a controller configured to select a test mode to be performed from a plurality of different test modes based on the intermediate measurement information, and control the sample preparation apparatus to prepare a test sample solution by using a second pretreatment test solution and a test reagent according to the selected test mode to be performed, wherein the second pretreatment test solution is prepared by the sample preparation apparatus using the test blood sample of the subject and a/the pretreatment reagent; and
a second test apparatus configured to test the test sample solution, so as to obtain a test result of the test blood sample for a target test item.

2. The sample analyzer of claim 1, **characterized in that** the pretreatment reagent for preparing the first pretreatment test solution comprises a hemolytic agent for lysing erythrocytes in the test blood sample to release hemoglobin, and the intermediate measurement information comprises a measurement value related to a hemoglobin concentration of the first pretreatment test solution.

3. The sample analyzer of claim 1, **characterized in that** the pretreatment reagent for preparing the first pretreatment test solution comprises a diluent for diluting the test blood sample to measure a hematocrit, and the intermediate measurement information comprises a measurement value related to a hematocrit of the first pretreatment test solution.

4. The sample analyzer of claim 2 or 3, **characterized in that** the target test item is a glycated hemoglobin test item, and the second test apparatus is further configured to measure glycated hemoglobin in the test blood sample.

5. The sample analyzer of claim 4, **characterized in that** the controller is further configured to:
control the sample preparation apparatus to prepare a first test sample solution by using the second pretreatment test solution and a first test reagent for the glycated hemoglobin test item according to the selected test mode to be performed, wherein the first test reagent comprises an enzyme reagent or an immunological reagent for measuring a hemoglobin content;
control the second test apparatus to test the first test sample solution, so as to obtain a hemoglobin content of the test blood sample;
control the sample preparation apparatus to add a second test reagent for the glycated hemoglobin test item to the first test sample solution, so as to prepare a second test sample solution, wherein the second test reagent comprises an enzyme reagent or an immunological reagent for measuring glycated hemoglobin;
control the second test apparatus to test the second test sample solution, so as to obtain a glycated hemoglobin content of the test blood sample; and
calculate a ratio of the glycated hemoglobin content to the hemoglobin content as a test result for the glycated hemoglobin test item.

6. The sample analyzer of any one of claims 1 to 5, **characterized in that** the first test apparatus is further configured to irradiate the first pretreatment test solution with a light of at least one wavelength, and obtain optical signals generated after the first pretreatment test solution is irradiated with the light, so as to obtain the intermediate measurement information based on the optical signals; or
the first test apparatus is further configured to allow the first pretreatment test solution to pass through for obtaining pulse measurement signals, so as to obtain the intermediate measurement information based on the pulse measurement signals.

7. The sample analyzer of any one of claims 1 to 6, **characterized in that** the first test apparatus and the second test apparatus are a same test apparatus.

8. The sample analyzer of any one of claims 1 to 7, **characterized in that** the first pretreatment test solution and the second pretreatment test solution are a same pretreatment test solution; or
the controller is further configured to: when the sample preparation apparatus is controlled to prepare the test sample solution by using the second pretreatment test solution and the test reagent according to the selected test mode to be performed, control the sample preparation apparatus to aspirate a blood sample of the subject again, prepare the second pretreatment test solution based on the blood sample aspirated again, and prepare the test sample solution based on the second pretreatment test solution prepared and the test reagent; or
the sample preparation apparatus is further configured to prepare a plurality of pretreatment test solutions by using the test blood sample and the pretreatment reagent, wherein the plurality of pretreatment test solutions are prepared differently from each other in terms of at least one of component, formula and dosage of the pretreatment reagent, and/or the plurality of pretreatment test solutions are prepared differently from each other in terms of dosage of the test blood sample;
the first test apparatus is further configured to test at least one first pretreatment test solution of the plurality of pretreatment test solutions, so as to obtain the intermediate measurement information of the first pretreatment test solution; and
the controller is further configured to: when the sample preparation apparatus is controlled to prepare the test sample solution by using the second pretreatment test solution and the test reagent according to the selected test mode to be performed, control the sample preparation apparatus to select one pretreatment test solution from the plurality of pretreatment test solutions as the second pretreatment test solution according to the selected test mode to be performed, and prepare the test sample solution by using the selected pretreatment test solution and the test reagent.

9. The sample analyzer of any one of claims 1 to 8, **characterized in that** the plurality of different test modes comprise at least two test modes, and effective test ranges of the at least two test modes are at least partially non-overlapping.

10. The sample analyzer of any one of claims 1 to 9, **characterized in that** the plurality of different test modes differ in at least one of the following aspects:
a dosage of at least one of the test blood sample, the pretreatment reagent and the test reagent used to prepare the test sample solution, or in the test sample solution, a proportion of at least one of the test blood sample, the pretreatment reagent and the test reagent used to prepare the test sample solution;
a component, a formula or a dosage of the pretreatment reagent used to prepare the test sample solution;
a type or a dosage of the test reagent used to prepare the test sample solution; and
a methodology for testing the test sample solution for the target test item.

11. The sample analyzer of any one of claims 1 to 10, **characterized in that** the pretreatment reagent for preparing the first pretreatment test solution comprises a hemolytic agent for lysing erythrocytes in the test blood sample to release hemoglobin, and the intermediate measurement information comprises a measurement value related to a hemoglobin concentration of the first pretreatment test solution; or the pretreatment reagent for preparing the first pretreatment test solution comprises a diluent for diluting the test blood sample to measure a hematocrit, and the intermediate measurement information comprises a measurement value related to a hematocrit of the first pretreatment test solution;
the target test item is a glycated hemoglobin test item, and the second test apparatus is further configured to measure glycated hemoglobin in the test blood sample; and
the controller is further configured to:
when the measurement value related to the hemoglobin concentration and/or the hematocrit of the first pretreatment test solution are/is within a preset range, select a first test mode as the test mode to be performed; and
when the measurement value related to the hemoglobin concentration and/or the hematocrit of the first pretreatment test solution are/is greater than an upper limit of the preset range, select a second test mode as the test mode to be performed, wherein, in the test sample solution, a proportion of the test blood sample or the second pretreatment test solution used to prepare the test sample solution under the first test mode is greater than a proportion of the test blood sample or the second pretreatment test solution used to prepare the test sample solution under the second test mode; and/or
when the measurement value related to the hemoglobin concentration and/or the hematocrit of the first pretreatment test solution are/is less than a lower limit of the preset range, select a third test mode as the test mode to be performed, wherein, in the test sample solution, the proportion of the test blood sample or the second pretreatment test solution used to prepare the test sample solution under the first test mode is less than a proportion of the test blood sample or the second pretreatment test solution used to prepare the test sample solution under the third test mode.

12. The sample analyzer of any one of claims 1 to 11, **characterized in that** the pretreatment reagent for preparing the first pretreatment test solution comprises a hemolytic agent for lysing erythrocytes in the test blood sample to release hemoglobin, and the intermediate measurement information comprises a measurement value related to a hemoglobin concentration of the first pretreatment test solution; or the pretreatment reagent for preparing the first pretreatment test solution comprises a diluent for diluting the test blood sample to measure a hematocrit, and the intermediate measurement information comprises a measurement value related to a hematocrit of the first pretreatment test solution;
the target test item is a glycated hemoglobin test item, and the second test apparatus is further configured to measure glycated hemoglobin in the test blood sample; and
the controller is further configured to:
when the measurement value related to the hemoglobin concentration and/or the hematocrit of the first pretreatment test solution are/is greater than a first preset threshold, select a fourth test mode as the test mode to be performed; and
when the measurement value related to the hemoglobin concentration and/or the hematocrit of the pretreatment test solution are/is less than the first preset threshold, select a fifth test mode as the test mode to be performed, wherein the test sample solution is prepared based on enzymatic assay for glycated hemoglobin under the fourth test mode, and the test sample solution is prepared based on turbidimetric inhibition immunoassay for glycated hemoglobin under the fifth test mode.

13. The sample analyzer of any one of claims 1 to 12, **characterized in that** the pretreatment reagent for preparing the first pretreatment test solution comprises a hemolytic agent for lysing erythrocytes in the test blood sample to release hemoglobin, and the intermediate measurement information comprises a measurement value related to an interferent in the first pretreatment test solution that interferes with the target test item;
the target test item is a glycated hemoglobin test item, and the second test apparatus is further configured to measure glycated hemoglobin in the test blood sample; and
the controller is further configured to:
when the measurement value related to the interferent in the first pretreatment test solution exceeds a second preset threshold, select a sixth test mode as the test mode to be performed, and control, according to the sixth test mode, the sample preparation apparatus to aspirate a blood sample of the subject again , prepare a new pretreatment test solution based on the blood sample aspirated again and a new pretreatment reagent, and prepare the test sample solution by using the new pretreatment test solution and the test reagent, wherein the new pretreatment reagent contains an anti-interference reagent for eliminating the interferent; and
when the measurement value related to the interferent in the first pretreatment test solution does not exceed the second preset threshold, select a seventh test mode as the test mode to be performed, and control, according to the seventh test mode, the sample preparation apparatus to prepare the test sample solution by using the test reagent and the second pretreatment test solution that is from the first pretreatment test solution tested by the first test apparatus.

14. A sample testing method, comprising:
aspirating a blood sample from a container containing a test blood sample of a subject, and performing pretreatment on the aspirated blood sample to obtain a first pretreatment test solution;
testing the first pretreatment test solution to obtain intermediate measurement information of the first pretreatment test solution;
selecting a test mode to be performed from a plurality of different test modes for a same target test item based on the intermediate measurement information of the first pretreatment test solution;
preparing a test sample solution by using a second pretreatment test solution and a test reagent according to the selected test mode to be performed, wherein the second pretreatment test solution is prepared by using the test blood sample of the subject and a pretreatment reagent; and
testing the test sample solution to obtain a test result for the test blood sample for the target test item.

15. The sample testing method of claim 14, **characterized in that** performing pretreatment on the aspirated blood sample to obtain a first pretreatment test solution comprises: mixing the aspirated blood sample with a/the pretreatment reagent to obtain the first pretreatment test solution,
wherein the pretreatment reagent for preparing the first pretreatment test solution comprises a hemolytic agent for lysing erythrocytes in the test blood sample to release hemoglobin, and the intermediate measurement information comprises a measurement value related to a hemoglobin concentration of the first pretreatment test solution; or the pretreatment reagent for preparing the first pretreatment test solution comprises a diluent for diluting the test blood sample to measure a hematocrit, and the intermediate measurement information comprises a measurement value related to a hematocrit of the first pretreatment test solution; or
performing pretreatment on the aspirated blood sample to obtain a first pretreatment test solution comprises: centrifuging the aspirated blood sample to obtain the first pretreatment test solution,
wherein the intermediate measurement information comprises a measurement value related to a hematocrit of the first pretreatment test solution, and the target test item is a glycated hemoglobin test item.

16. The sample testing method of claim 15, **characterized in that** the first pretreatment test solution and the second pretreatment test solution are a same pretreatment test solution; or
preparing the test sample solution comprises: aspirating a blood sample of the subj ect again, preparing the second pretreatment test solution based on the blood sample aspirated again, and preparing the test sample solution based on the second pretreatment test solution prepared and the test reagent.
